Europäisches Patentamt

European Patent Office

Office européen des brevets

(19)

(11) **EP 1 473 010 A1**

(12) **EUROPEAN PATENT APPLICATION**
published in accordance with Art. 158(3) EPC

(43) Date of publication:
**03.11.2004 Bulletin 2004/45**

(51) Int Cl.$^7$: **A61F 13/53**

(21) Application number: **03703028.5**

(22) Date of filing: **23.01.2003**

(86) International application number:
**PCT/JP2003/000584**

(87) International publication number:
**WO 2003/065958 (14.08.2003 Gazette 2003/33)**

(84) Designated Contracting States:
**AT BE BG CH CY CZ DE DK EE ES FI FR GB GR HU IE IT LI LU MC NL PT SE TR**

(30) Priority: **04.02.2002 JP 2002026383**

(71) Applicant: **Nippon Shokubai Co., Ltd.**
**Osaka-shi, Osaka 541-0043 (JP)**

(72) Inventors:
• **NAGASUNA, Kinya**
**Kitakatsuragi-gun, Nara 636-0091 (JP)**
• **IMURA, Motohiro**
**Takatsuki-shi, Osaka 569-0854 (JP)**
• **KADONAGA, Kenji**
**Takatsuki-shi, Osaka 569-0854 (JP)**
• **INOUE, Hiroki**
**Kyoto-shi, Kyoto 616-0027 (JP)**
• **SASABE, Masazumi**
**Kakogawa-shi, Hyogo 675-0122 (JP)**
• **MINAMI, Kenji**
**Otsu-shi, Shiga 520-0227 (JP)**

(74) Representative: **Glawe. Delfs. Moll**
**Patentanwälte**
**Postfach 26 01 62**
**80058 München (DE)**

(54) **ABSORPTIVE MATERIAL, METHOD FOR PRODUCING THE SAME AND ABSORPTIVE ARTICLE USING THE SAME**

(57)    An object of the present invention is to provide an absorbent structure, its production process, and an absorbent article comprising the absorbent structure, wherein, in the absorbent structure, a water-absorbent resin is fixed, and the restrictions, due to this fixation, on the swelling are small, and the absorbent structure is excellent in absorption properties and usable favorably for thin type absorbent articles in which the water-absorbent resin is used in an increased amount. As a means of achieving this object, the absorbent structure according to the present invention comprises a base material, a water-absorbent resin layer, and a hot-melt adhesive, wherein the water-absorbent resin layer includes a water-absorbent resin as an essential material and is fixed to the base material with the hot-melt adhesive; with the absorbent structure being characterized by: being a multi-layered structure including at least the following three layers: a layer of the base material; the water-absorbent resin layer; and the hot-melt adhesive; and displaying an average space ratio of 30 to 70 % and an average space radius of 100 to 300 μm during saturation-swelling without load.

Fig. 3

31      32      33

EP 1 473 010 A1

Printed by Jouve, 75001 PARIS (FR)

**Description**

TECHNICAL FIELD

The present invention relates to an absorbent structure, its production process, and an absorbent article, wherein the absorbent structure is used favorably for sanitary materials such as disposable diapers, sanitary napkins, and so-called incontinent pads.

BACKGROUND ART

At present, for sanitary materials such as disposable diapers, sanitary napkins, and so-called incontinent pads, there are widely utilized, as their constituent materials, hydrophilic fibers (e.g. pulp) and water-absorbent resins (which are water-swellable crosslinked polymers obtained using such as acrylic acid (and/or its salts) as main raw materials) for the purpose of absorption of body fluids. In recent years, as to these sanitary materials such as disposable diapers and sanitary napkins, their high functionalization and thinning are making progress, so there is a tendency toward increases in the amount of the water-absorbent resin as used per piece of sanitary material and in mass % of the water-absorbent resin relative to a whole absorbent structure consisting of such as the water-absorbent resin and the hydrophilic fibers. Specifically, the ratio of the water-absorbent resin in the absorbent structure is raised by decreasing the amount of the hydrophilic fibers (which have a small bulk density) and increasing the amount of the water-absorbent resin (which has excellent water absorbency and a large bulk density) as used. Thereby the thinning of the sanitary materials is aimed at without lowering the water absorption quantity.

However, the sanitary materials, in which the ratio of the hydrophilic fibers has been decreased and that of the water-absorbent resin has been increased in the above way, are in a favorable direction from the viewpoint of simple storage of liquids, but rather involve problems in the case of consideration of distribution and diffusion of the liquids under circumstances of actual use of diapers. The large amount of water-absorbent resin becomes a soft gel due to water absorption to cause a phenomenon which is called "gel-blocking" that much hinders the diffusion of the liquids. In order to avoid such problems to maintain absorption properties of the absorbent structure, the ratios of the hydrophilic fibers and the water-absorbent resin are axiomatically limited, so a limit occurs also to the thinning of the sanitary materials.

As means of preventing the gel-blocking, there are proposed such as: a method in which two kinds of water-absorbent resins different as to water absorption performance are used (JP-A-252307/2001 (Kokai) (Oji Paper-Manufacturing Co., Ltd.)); a method in which a composition containing a cationic ion-exchange hydrogel-forming polymer and an anionic ion-exchange hydrogel-forming polymer is used (WO 98037149 (P&G)); a method in which a water-absorbent resin having a high surface-crosslinking density is used (JP-A-057010/1994 (Kokai) (Kao Corporation)); and a method in which a mixture of a water-absorbent resin and a thermoplastic resin is foam-extruded to form a sheet (WO 01/64153A1 (Paul Hartman)). However, they have problems such that the absorption properties are unsatisfactory as the absorbent structure having a high water-absorbent resin concentration or that the cost is high.

In addition, because the relative amount of the hydrophilic fibers which serve to fix the water-absorbent resin is lowered, there occur problems such that the particles of the water-absorbent resin become localized before the use or move during the use. The water-absorbent resin, in this way, gets out of the designed position in the absorbent article to thereby cause its deformation, so that there occur problems of leading to leakage without contact of subsequently excreted liquids (e.g. urine) with the water-absorbent resin in the absorbent article.

As to techniques for fixing the water-absorbent resin in the absorbent article, many studies have hitherto been made, and there are known such as: a method in which an absorbent structure is subjected to an embossing treatment; a method in which an absorbent structure including a water-absorbent resin and hydrophilic fibers is heat-fused by causing the absorbent structure to contain thermoplastic binder fibers; a method in which an absorbent structure including a water-absorbent resin and hydrophilic fibers is heat-fused by causing the absorbent structure to contain a synthetic resin having a high ratio of recovery from a warp (JP-A-118114/1998 (Kokai), JP-A-118115/1998 (Kokai)); a method in which surfaces of a water-absorbent resin having an anionic group are coated with a cationic polymer so as to fix particles to each other by adhesion when swelling them (JP-A-031362/1993 (Kokai), JP-A-000370/1994 (Kokai)); a method in which a water-absorbent resin and hydrophilic fibers are fixed to each other with an emulsion binder; and a method in which a water-absorbent resin is fixed to a base material with a hot-melt adhesive (JP-A-238161/2000 (Kokai), JP-A-510447/1998 (Kohyo)).

However, in the case where the water-absorbent resin is fixed to the base material, there occurs a problem of restrictions on the swelling of the water-absorbent resin due to restraint by the binder force. Particularly in the case where such as the water-absorbent resin and the hydrophilic fibers are fixed with the thermoplastic binder or the emulsion, there are problems such that the absorption ability which the water-absorbent resin intrinsically possess cannot sufficiently be displayed.

As techniques for easing the restrictions, due to such fixation, on the swelling of the water-absorbent resin, there are also known: an absorbent composite such that a water-absorbent resin, of which a part is wrapped and held inside a bulky nonwoven fabric and of which surfaces are coated with fine cellulose fibers, is further coated with net-shaped hot-melt fibers from outer surfaces of the resin (JP-A-096654/2001 (Kokai)); and an absorbent composite sheet such that a water-absorbent resin, of which a part is wrapped and held inside a bulky nonwoven fabric and of which a part is exposed from surfaces, is coated with hot-melt fibers from outer surfaces of the resin wherein the hot-melt fibers are of the shape of a double net different as to net mesh (JP-A-171027/2001 (Kokai)).

Furthermore, the fixation of the water-absorbent resin has another problem of a change of absorption properties due to the fixation besides the restrictions on the swelling as brought about by the above fixation means. As the water absorption properties of the water-absorbent resin, there is a water absorption property based on the capillary force of openings between particles, wherein this property is a capillary absorption capacity as disclosed in such as the specifications of Japanese Patent Application Nos. 2002-072476 and 2001-375375, in addition to those which have hitherto been well known, such as water absorption capacity, water absorption rate, absorption capacity under load, diffusing absorption capacity under load, and liquid permeability of swollen gel.

The above water absorption properties, such as capillary absorption capacity, of the water-absorbent resin have turned out to be influenced greatly by the hitherto existing fixation means. Specifically, there are many cases where, even if a high-performance water-absorbent resin is used, the water absorption properties of the original water-absorbent resin cannot be reflected in the absorption properties of the absorbent structure as obtained by the fixation. JP-A-096654/2001 (Kokai) also discloses that, if only a certain performance as required at the minimum is satisfied, it is unnecessary to much bother about performances of a usable water-absorbent resin. In a state of the formed absorbent structure, there hardly occur differences in absorption properties between water-absorbent resins, and it is therefore difficult to differentiate the absorbent structures.

In addition, as to absorbent articles such as thin type sanitary materials having an increased water-absorbent resin concentration, the amount of the water-absorbent resin as used is increased and, depending on its arrangement position, there is a case where, after absorbing a liquid, the resin swells to become considerably bulky. There is also a problem such that the pressure as put on bodies by this bulkiness tends to increase in proportion as the water-absorbent resin is strongly fixed.

DISCLOSURE OF THE INVENTION

OBJECTS OF THE INVENTION

Thus, an object of the present invention is to provide an absorbent structure, its production process, and an absorbent article comprising the absorbent structure, wherein the absorbent structure has a high water-absorbent resin content and is excellent in the absorption quantity, the absorption rate, and the liquid-diffusing ability, and solves the above problems and is used for absorbent articles such as thin type sanitary materials, and wherein, in the absorbent structure, a water-absorbent resin is well fixed to a base material, and the restrictions, due to this fixation, on the swelling are small, and the absorbent structure is excellent also in absorption properties such as capillary absorption capacity and realizes the thinning and weight-lightening so as to be used most suitably for absorbent articles.

In addition, another object of the present invention is to provide an absorbent structure, its production process, and an absorbent article comprising the absorbent structure, wherein the absorbent structure avoids the problem such that, as to absorbent articles such as thin type sanitary materials having an increased water-absorbent resin concentration (ratio of the resin as used), the water-absorbent resin bulkily swells to put the pressure on bodies with the progress of the absorption of liquids, and wherein the absorbent structure does not greatly change such as thickness of crotch zone even if the liquid absorption quantity increases.

SUMMARY OF THE INVENTION

The present inventors diligently studied to achieve the above objects.

As a result, they have directed their attention to an absorbent structure which comprises a base material, a water-absorbent resin layer, and a hot-melt adhesive, wherein the water-absorbent resin layer includes a water-absorbent resin as an essential material (hereinafter this layer may be referred to simply as water-absorbent resin layer) and is fixed to the base material with the hot-melt adhesive; and then the inventors have found out that: if the absorbent structure has a multi-layered structure including a pile of at least the following three layers: a layer of the base material; the water-absorbent resin layer; and the hot-melt adhesive; then the water-absorbent resin layer can be fixed to the base material effectively with a small amount of the hot-melt adhesive, and also, even in the case where the ratio of the water-absorbent resin as used is considerably high, there can be obtained an absorbent structure possessing such unprecedentedly excellent absorbency as to satisfy specific ranges in the average space ratio and average space radius during saturation-swelling or a specific range in the capillary absorption capacity.

In addition, the present inventors have directed their attention also to an absorbent structure which comprises a water-absorbent resin as an essential material, and then they have found out that: if not less than 50 mass % of the water-absorbent resin (water-absorbent resin particles particles) as used satisfies specific ranges in the average space ratio and average space radius as to spaces between particles during saturation-swelling, then there can be obtained an absorbent structure which can satisfy specific ranges in the average space ratio and average space radius during saturation-swelling or a specific range in the capillary absorption capacity and therefore possesses unprecedentedly excellent absorbency also in such as properties of permeating, diffusing, and sucking up liquids, even in the case where the ratio of the water-absorbent resin as used in the absorbent structure has been made considerably high.

Moreover, the present inventors have found out that: as to the aforementioned absorbent structure (in which the water-absorbent resin layer is fixed to the base material with the hot-melt adhesive), if the kind of the hot-melt adhesive and/or its supply method are specified, then there can be obtained an absorbent structure which is such that the restrictions, due to the fixation of the water-absorbent resin layer, on the swelling is greatly eased, and which is excellent in fixability of the water-absorbent resin layer in a dry state before use; and further, the aforementioned multi-layered structure makes it possible to provide a thin type absorbent structure of which the absorbing portion is prevented from becoming bulky, because the water-absorbent resin layer as fixed to the base material is released from the fixing force little by little with the progress of the swelling of the water-absorbent resin layer to thus become mobile.

Also, the present inventors have further found out that the use of these absorbent structures make it possible to provide a thin type absorbent article which displays absorbency such as very excellent liquid-absorbing ability.

In the above way, the present invention has been completed.

That is to say, the above objects are achieved by the following (1) to (18).

(1) An absorbent structure, which comprises a base material, a water-absorbent resin layer, and a hot-melt adhesive, wherein the water-absorbent resin layer includes a water-absorbent resin as an essential material and is fixed to the base material with the hot-melt adhesive; with the absorbent structure being characterized by: being a multi-layered structure including at least the following three layers: a layer of the base material; the water-absorbent resin layer; and the hot-melt adhesive; and displaying an average space ratio of 30 to 70 % and an average space radius of 100 to 300 $\mu$m during saturation-swelling with a physiological saline (a 0.9 mass % aqueous NaCl solution) without load.

(2) An absorbent structure, which comprises a base material, a water-absorbent resin layer, and a hot-melt adhesive, wherein the water-absorbent resin layer includes a water-absorbent resin as an essential material and is fixed to the base material with the hot-melt adhesive; with the absorbent structure being characterized by: being a multi-layered structure including at least the following three layers: a layer of the base material; the water-absorbent resin layer; and the hot-melt adhesive; and displaying an average space ratio of 20 to 50 % and an average space radius of 50 to 130 $\mu$m during saturation-swelling with a physiological saline (a 0.9 mass % aqueous NaCl solution) under a load of 2.07 kPa (0.3 psi).

(3) An absorbent structure, which comprises a base material, a water-absorbent resin layer, and a hot-melt adhesive, wherein the water-absorbent resin layer includes a water-absorbent resin as an essential material and is fixed to the base material with the hot-melt adhesive; with the absorbent structure being characterized by: being a multi-layered structure including at least the following three layers: a layer of the base material; the water-absorbent resin layer; and the hot-melt adhesive; and displaying a capillary absorption capacity of not less than 10 (g/g) at a height of 20 cm.

(4) An absorbent structure according to any one of (1) to (3) aforementioned, wherein the hot-melt adhesive is in contact with the water-absorbent resin layer in the shape of a finely fibrous net.

(5) An absorbent structure, which comprises a base material, a water-absorbent resin layer, and a hot-melt adhesive, wherein the water-absorbent resin layer includes a water-absorbent resin as an essential material and is fixed to the base material with the hot-melt adhesive; with the absorbent structure being characterized by being a multi-layered structure including at least the following four layers: a layer of the base material; the water-absorbent resin layer; the hot-melt adhesive; and a member adherent to the hot-melt adhesive.

(6) An absorbent structure according to (5) aforementioned, wherein the member adherent to the hot-melt adhesive is an anti-sticking material.

(7) An absorbent structure according to (5) aforementioned, wherein the member adherent to the hot-melt adhesive is a hydrophilic release material.

(8) An absorbent structure, which comprises a base material, a water-absorbent resin layer, and a hot-melt adhesive, wherein the water-absorbent resin layer includes a water-absorbent resin as an essential material and is fixed to the base material with the hot-melt adhesive; with the absorbent structure being: characterized by being a multi-layered structure including at least the following three layers: a layer of the base material; the water-absorbent resin layer; and the hot-melt adhesive; and further characterized in that the mobility of the water-absorbent resin as contained in the water-absorbent resin layer increases in the absorbent structure in proportion as the water-absorbent resin layer absorbs a liquid to increase its swelling ratio.

(9) An absorbent structure, which comprises a water-absorbent resin as an essential material; with the absorbent structure being characterized in that the water-absorbent resin exists in a ratio of not less than 50 mass % in the absorbent structure, wherein the water-absorbent resin displays an average space ratio of 45 to 70 % and an average space radius of 100 to 200 μm as to spaces between particles during saturation-swelling with a physiological saline (a 0.9 mass % aqueous NaCl solution) without load, and further displays a capillary absorption capacity of at least 20 g/g.

(10) An absorbent structure, which comprises a water-absorbent resin as an essential material; with the absorbent structure being characterized in that the water-absorbent resin exists in a ratio of not less than 50 mass % in the absorbent structure, wherein the water-absorbent resin displays an average space ratio of 45 to 70 % and an average space radius of 100 to 200 μm as to spaces between particles during saturation-swelling with a physiological saline (a 0.9 mass % aqueous NaCl solution) without load, and further displays an average space ratio of 20 to 50 % and an average space radius of 50 to 85 μm as to spaces between particles during saturation-swelling with a physiological saline (a 0.9 mass % aqueous NaCl solution) under a load of 2.07 kPa (0.3 psi).

(11) An absorbent article, which comprises the absorbent structure as recited in any one of (1) to (10) aforementioned.

(12) A production process for an absorbent structure, which comprises the steps of: supplying a water-absorbent resin as an essential material to a base material, thereby forming a water-absorbent resin layer; and supplying a hot-melt adhesive to the water-absorbent resin layer, thereby fixing the water-absorbent resin layer to the base material; with the production process being characterized by using, as the water-absorbent resin, a water-absorbent resin which displays an average space ratio of 30 to 70 % and an average space radius of 100 to 300 μm as to spaces between particles during saturation-swelling with a physiological saline (a 0.9 mass % aqueous NaCl solution) without load.

(13) A production process for an absorbent structure, which comprises the steps of: supplying a water-absorbent resin as an essential material to a base material, thereby forming a water-absorbent resin layer; and supplying a hot-melt adhesive to the water-absorbent resin layer, thereby fixing the water-absorbent resin layer to the base material; with the production process being characterized by using, as the water-absorbent resin, a water-absorbent resin which displays an average space ratio of 20 to 50 % and an average space radius of 50 to 130 μm as to spaces between particles during saturation-swelling with a physiological saline (a 0.9 mass % aqueous NaCl solution) under a load of 2.07 kPa (0.3 psi).

(14) A production process for an absorbent structure according to (12) or (13) aforementioned, wherein the hot-melt adhesive is brought into contact with the water-absorbent resin layer in the shape of a finely fibrous net.

(15) A production process for an absorbent structure, which comprises the steps of: supplying a water-absorbent resin as an essential material to a base material, thereby forming a water-absorbent resin layer; and supplying a hot-melt adhesive to the water-absorbent resin layer, thereby fixing the water-absorbent resin layer to the base material; with the production process being characterized in that: the hot-melt adhesive has a melt viscosity of 700 to 40,000 mPa·s at 160 °C, and is dispersed into the shape of a web of fine fibers by a non-contact-type melt-blow-spraying method, and the dispersed hot-melt adhesive has an amount of 0.1 to 50 g/m$^2$, an average fiber diameter of 5 to 150 μm, a number-average diameter of 10 to 500 μm as to intervals between fibers, and an elongation of not less than 200 % as to fibers at 25 °C.

(16) A production process for an absorbent structure according to (15) aforementioned, wherein: the melt viscosity is in the range of 2,000 to 7,000 mPa·s, and the elongation is not less than 500 %.

(17) A production process for an absorbent structure according to (15) or (16) aforementioned, wherein the water-absorbent resin has an average particle diameter of 50 to 600 μm.

(18) A production process for an absorbent structure, which comprises the steps of: supplying a water-absorbent resin as an essential material to a base material, thereby forming a water-absorbent resin layer; and supplying a hot-melt adhesive to the water-absorbent resin layer, thereby fixing the water-absorbent resin layer to the base material; with the production process being characterized by further comprising the step of temporarily fixing the water-absorbent resin layer to the base material with a temporarily fixing agent before the step of supplying the hot-melt adhesive.

BRIEF DESCRIPTION OF THE DRAWINGS

Fig. 1 is a schematic sectional view of a measurement apparatus as used for measurement to determine the average space ratio, the average space radius, and the capillary absorption capacity.

Fig. 2 is a schematic sectional view of a measurement apparatus as used for measurement of the absorption capacity of a water-absorbent resin under a load.

Fig. 3 is a schematic view of a model absorbent article (for a plane test).

Fig. 4 is a schematic sectional view of a model absorbent article (for an inclination test).

Fig. 5 is a schematic view of an apparatus as used for an L-character test as used for evaluation of a water-absorbent article.

An explanation of the symbols is as follows.

1: Porous glass plate

2: Glass filter

3: Conduit

4: Liquid storage container

5: Supporting ring

6: Physiological saline (0.9 mass % aqueous NaCl solution)

7: Balance

8: Stand

9: Specimen to be measured (water-absorbent resin or absorbent structure)

10: Load (0.41 kPa (0.06 psi))

11: Air-intake pipe

12: Conduit

13: Glass filter

14: Physiological saline (0.9 mass % aqueous NaCl solution)

15: Liquid storage container

16: Balance

17: Filter paper

18: Metal gauze

19: Plastic cylinder

20: Load (2.07 kPa (0.3 psi))

21: Load (4.83 kPa (0.7 psi))

31: Liquid-permeable polyester nonwoven fabric

32: Water-absorbent resin layer which is fixed with a hot-melt adhesive and to which a hydrophilic release agent is attached

33: Heatlon paper (base material)

41: Liquid-permeable surface material (top sheet)

42: Water-absorbent resin

43: Diffusing paper

44: Liquid-impermeable material (back sheet)

45: Solvent type pressure sensitive adhesive

46: Double coated tape

47: Side gather

48: Injection position

49: Model absorbent article

50: Weight

51: Absorbent structure (water-absorbent resin)

52: Inclined-plane stand

DETAILED DESCRIPTION OF THE INVENTION

Hereinafter, detailed descriptions are given about the absorbent structure, its production process, and the absorbent article comprising the absorbent structure, according to the present invention. However, the scope of the present invention is not bound to these descriptions. And other than the following illustrations can also be carried out in the form of appropriate modifications of the following illustrations within the scope not departing from the spirit of the present invention.

(1) Constitution of absorbent structure (first absorbent structure):

The absorbent structure, according to the present invention, comprises at least a water-absorbent resin as an essential material and, if necessary, comprises a water-absorbent resin and a base material.

Examples of favorable modes for carrying out the absorbent structure according to the present invention include: an absorbent structure which comprises at least a base material (base material layer), a water-absorbent resin layer including a water-absorbent resin as an essential material (water-absorbent resin layer including the water-absorbent resin and, if necessary, further including another material), and a hot-melt adhesive, wherein the water-absorbent resin layer is fixed to the above base material with the hot-melt adhesive; with the absorbent structure having (or being) a multi-layered structure including either a pile of at least these three kinds or at least these three layers, namely, the base material (base material layer), the water-absorbent resin layer, and the hot-melt adhesive; or with the absorbent structure having (or being) a multi-layered structure including either a pile of the following four kinds or the following four layers, namely, the base material (base material layer), the water-absorbent resin layer, the hot-melt adhesive, and a member adherent to the hot-melt adhesive. However, the latter (having the multi-layered structure including either the pile of the four kinds or the four layers) is preferable. In the case where the three or four layers are included in the above, the hot-melt adhesive and the member adherent to the hot-melt adhesive are defined as layered ones. In addition, as to the above three kinds or layers or four kinds or layers of constituent materials, it is herein specified that it is enough that they are piled or layered in order of their recitation as above. In the case where another constituent material is further used, it may be used in the form piled in any portion within the range not changing the order as to the above three kinds or layers or four kinds or layers of constituent materials. The above base material (base material layer) may be either a single layer or a multiple layer of at least two members.

The absorbent structure, according to the present invention, is formed so as to satisfy properties such as average space ratio, average space radius, and capillary absorption capacity by appropriately selecting such as: kinds, properties, and performances of constituent materials (e.g. water-absorbent resin, hot-melt adhesive); and method for the fixation with the hot-melt adhesive. The absorbent structure, according to the present invention, can be in the form of a sheet having the above multi-layered structure or in the form of a cylinder or three-dimensionally molded product having the above multi-layered structure, and has a function to absorb liquids to store them.

Hereinafter, in the present specification, the absorbent structure as recited in the present item (1), in which the water-absorbent resin layer is fixed to the base material with the hot-melt adhesive, may be referred to as "first absorbent structure". In addition, unless otherwise noted, the statements of from the present item (1) to the below-mentioned item (7) are made as descriptions about the first absorbent structure.

The water-absorbent resin layer may comprise the water-absorbent resin alone, but, if necessary, may further contain another material. Examples of this other material, which gets contained if necessary, include those which enhance water absorption performances of the water-absorbent resin layer or give it functions, and specific examples thereof include: water-insoluble finely-particulate inorganic powders, such as silicon dioxide, titanium dioxide, aluminum oxide, magnesium oxide, zinc oxide, talc, calcium phosphate, barium phosphate, silicic acid or its salts, clay, diatomite, zeolite, bentonite, kaolin, hydrotalcite, and salts (e.g. activated clay); fibers, such as cellulose fibers obtained from wood (e.g. mechanical pulp, chemical pulp, semichemical pulp, dissolving pulp), regenerated fibers (e.g. rayon, acetate), and synthetic fibers (e.g. polyamides, polyesters, polyolefins); and besides, deodorants, perfumes, antibacterial agents, cationic polymer compounds (e.g. polyamines), foaming agents, pigments, dyes, hydrophilic short fibers, manures, oxidizing agents, reducing agents, and water.

In the water-absorbent resin layer, the water-absorbent resin is contained in such a concentration that the water-absorbent resin can be adjacent to each other when it swells by absorption of water, for the purpose of obtaining an absorbent structure which is a thin type and has high water absorbency. The water-absorbent resin content of the water-absorbent resin layer is not less than 70 mass %, favorably not less than 80 mass %, more favorably not less than 90 mass %.

The amount of the water-absorbent resin layer is usually in the range of about 100 to about 2,000 g/m$^2$. In the case where this amount is smaller than 250 g/m$^2$, the saturated absorption quantity of the absorbent structure is so small that there is seen a tendency for the comfortableness to deteriorate (for the feel of dryness to be so inferior that the wet-back quantity increases) when the absorbent structure is used for body-fluid-absorbent articles. The amount of the water-absorbent resin layer is favorably in the range of about 200 to about 1,500 g/m$^2$, more favorably about 300 to about 800 g/m$^2$.

(2) Average space ratio and average space radius of absorbent structure:

The absorbent structure according to the present invention, in which the water-absorbent resin layer is fixed by supplying the water-absorbent resin layer and the hot-melt adhesive to the base material, is ① an absorbent structure which displays an average space ratio of 30 to 70 % and an average space radius of 100 to 300 μm during saturation-swelling without load or ② an absorbent structure which displays an average space ratio of 20 to 50 % and an average space radius of 50 to 130 μm during saturation-swelling under a load of 2.07 kPa (0.3 psi).

The absorbent structure, according to the present invention, is usually obtained by fixing the water-absorbent resin layer to the base material with hot-melt fibers wherein the water-absorbent resin layer includes a water-absorbent resin (water-absorbent resin particles) which displays an average space ratio of 30 to 70 % and an average space radius of 100 to 300 μm as to spaces between particles during saturation-swelling without load or a water-absorbent resin (water-absorbent resin particles) which displays an average space ratio of 20 to 50 % and an average space radius of 50 to 130 μm as to spaces between particles during saturation-swelling under a load of 2.07 kPa (0.3 psi). However, even if the average space ratio and average space radius as to spaces between particles of the water-absorbent resin are not necessarily in the above ranges, the absorbent structure having the average space ratio and average space radius as to spaces between particles, according to the present invention, can be obtained by selecting such as the base material, the method for supply of the hot-melt and the kind of the hot-melt and thus forming and adjusting the spaces between particles with the hot-melt adhesive fibers.

As to an absorbent structure which displays an average space ratio of more than 70 % during saturation-swelling without load, the average space ratio of the absorbent structure tends to be too large, and the sucking-up property of the absorbent structure is deteriorated to such an extent that there is a tendency toward the increase of such as wet-back quantity without good absorption of liquids by the absorbent structure. As to an absorbent structure which displays an average space ratio of less than 30 % during saturation-swelling without load, liquids cannot well permeate the absorbent structure, therefore the absorption rate is so slow that there is a possibility that the leakage may occur from the initial stage. A favorable absorbent structure displays an average space ratio of 35 to 45 % during saturation-swelling without load.

In addition, as to an absorbent structure which displays an average space radius of greater than 300 μm during saturation-swelling without load, the sucking-up property of the absorbent structure is deteriorated to such an extent that, when the absorbent structure is further used for absorbent articles such as disposable diapers, its ability to suck liquids from other members such as pulp is deteriorated so greatly as to have a feel of much wetness. As to an absorbent structure which displays an average space radius of smaller than 100 μm during saturation-swelling without load, liquids cannot well permeate the absorbent structure, therefore the absorption rate is so slow that there is a possibility that the leakage may occur from the initial stage. A favorable absorbent structure displays an average space radius of 120 to 250 μm during saturation-swelling without load.

As to an absorbent structure which displays an average space ratio of more than 50 % during saturation-swelling under a load of 2.07 kPa (0.3 psi), the average space ratio of the absorbent structure tends to be too large, and the sucking-up property of the absorbent structure is deteriorated under the load to such an extent that there is a tendency toward the increase of such as wet-back quantity without good absorption of liquids by the absorbent structure. As to an absorbent structure which displays an average space ratio of less than 20 % during saturation-swelling under a load of 2.07 kPa (0.3 psi), similarly, liquids cannot well permeate the absorbent structure, therefore the absorption rate is so slow under the load that there is a possibility that the leakage may occur from the initial stage. A favorable absorbent structure displays an average space ratio of 25 to 30 % during saturation-swelling under a load of 2.07 kPa (0.3 psi).

In addition, as to an absorbent structure which displays an average space radius of greater than 130 μm during saturation-swelling under a load of 2.07 kPa (0.3 psi), similarly, the sucking-up property of the absorbent structure is deteriorated under the load to such an extent that, when the absorbent structure is further used for absorbent articles such as disposable diapers, its ability to suck liquids from other liquid-acquiring base materials such as pulp is deteriorated so greatly as to have a feel of much wetness. As to an absorbent structure which displays an average space radius of smaller than 50 μm during saturation-swelling under a load of 2.07 kPa (0.3 psi), liquids cannot well permeate the absorbent structure, therefore the absorption rate is so slow that there is a possibility that the leakage may occur from the initial stage. A favorable absorbent structure displays an average space radius of 50 to 70 μm during saturation-swelling under a load of 2.07 kPa (0.3 psi).

It is enough that the absorbent structure, according to the present invention, satisfies the above requirement ① or ②, but it is favorable that this absorbent structure satisfies both the above requirements ① and ②. In the case where only one of these requirements is satisfied, there is a possibility that, depending on conditions for the use of the absorbent structure, its absorption properties may not well be displayed.

(3) Capillary absorption capacity of absorbent structure:

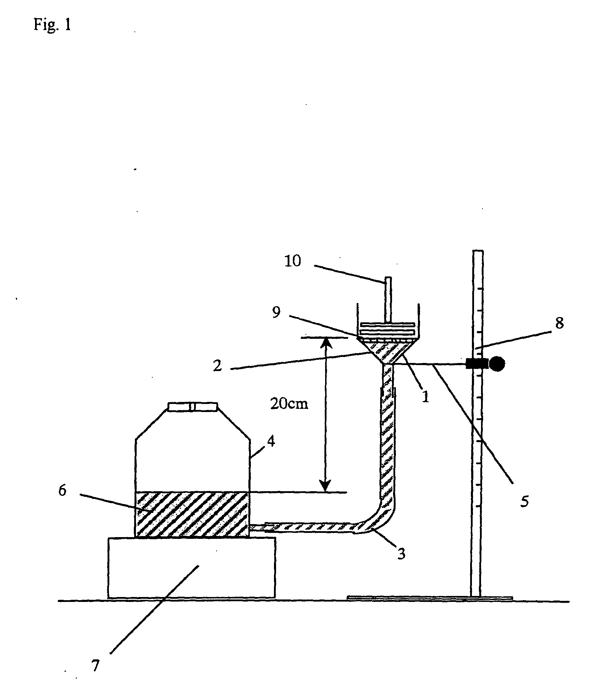

In the present invention, the capillary absorption capacity, which is used to evaluate the capillary absorption ability of the absorbent structure having the fixed water-absorbent resin layer, is: an application of a method as conventionally used to measure the absorption force of a material that sucks up and absorbs liquids by a capillary phenomenon such as paper and pulp; and is a method for evaluating the capillary absorption force and the liquid-sucking-up ability of a specimen by measuring the quantity of a liquid as absorbed per unit mass of the specimen under conditions where the liquid absorption position is changed to various heights with the below-mentioned apparatus of Fig. 1. As to a specific method for measuring the capillary absorption capacity which is the capillary suction ability in the present invention, it is described in detail in the below-mentioned examples of some preferred embodiments, but measurements methods based on the same principles are disclosed also in such as: Textile Research Journal, Vol. 57, p. 356 (1967); "Absorbency" (Chatterjee, Textile Science and Technology, Vol. 7, 1985); JP-A-052349/1996 (Kokai); and WO 99/47184.

In the present invention, there is measured the absorption capacity of a liquid as absorbed by the absorbent structure within a predetermined time under conditions where a height H1 of the liquid absorption position is stated in a position higher than a height H2 of the surface of the liquid as contained in a liquid storage container. By its value, there can rightly be evaluated the ability of the absorbent structure to absorb the liquid from other members such as pulp. For enhancing its accuracy, it is favorable to carry out the evaluation at a difference of 20 to 60 cm in altitude between the height H1 of the liquid absorption position and the height H2 of the surface of the liquid as contained in the liquid storage container. In the present invention, there is evaluated the capillary absorption capacity at 20 cm as set by making a difference of 20 cm in altitude between the liquid absorption position and the surface of the liquid as contained in the liquid storage container.

The present inventors directed their attention to the ability referred to as capillary absorption ability as to the absorbent structure having the fixed water-absorbent resin layer, and studied about this ability. As a result, they have found out that, even if an identical water-absorbent resin is used, the capillary absorption ability of the absorbent structure greatly changes according to: the kind of the base material to which the water-absorbent resin is fixed; the kind of the binder used for the fixation; and the method for the fixation. The absorbent structure, according to the present invention, is evaluated by the above capillary absorption capacity at 20 cm and, in the present invention, its value is favorably not less than 10 (g/g). In the case where the capillary absorption capacity at 20 cm is less than 10 (g/g), there is a possibility that the absorbent structure cannot absorb the liquid from other members, therefore absorbent articles comprising this absorbent structure are inferior in the feel of dryness. The capillary absorption capacity of the absorbent structure at 20 cm is favorably not less than 15 (g/g), more favorably not less than 20 (g/g), most favorably not less than 25 (g/g).

Even if the capillary absorption capacity of the water-absorbent resin alone at 20 cm is not less than 10 (g/g), there are many cases where, as mentioned above, dependently on the kind of the base material and the method for the fixation, it is impossible to maintain this value. The absorbent structure having the capillary absorption capacity of not less than 10 (g/g) can be achieved by: controlling, within the ranges according to the present invention, the average space ratio and average space radius of the absorbent structure during saturation-swelling without load or the average space ratio and average space radius of the absorbent structure during saturation-swelling under a load of 2.07 kPa (0.3 psi); and further, using a resin having a capillary absorption capacity in a predetermined range as the water-absorbent resin.

(4) Base material (fixation base material):

In the present invention, the base material to which the water-absorbent resin layer is fixed by supplying the water-absorbent resin layer and the hot-melt adhesive (which may be referred to as fixation base material) has a structure such that the water-absorbent resin layer can be present on its surface or in its inside, and is favorably a sheet-shaped one.

Examples of the fixation base material usable in the present invention include: paper, pulp, sheets of cellulose fibers (e.g. rayon, cellulose acetate, nitrocellulose, crosslinked cellulose), nonwoven fabrics, porous polymer sheets obtained by polymerization of high internal phase emulsion (HIPE), foam sheets comprising synthetic polymers (e.g. polyurethane, polystyrene, polyethylene, polypropylene, polyester, polyvinyl alcohol, butadiene-styrene rubber (SBR), nitrile-butadiene rubber); fiber aggregates formed by adhesion or biding of synthetic fibers (e.g. polyethylene, polypropylene, polyethylene terephthalate, nylon), and fiber aggregates formed by press-attaching, adhesion, or biding of hydrophilic fibers (e.g. polyamide fibers). Favorable ones are paper and nonwoven fabrics. Changes of such as opening ratios, thicknesses, and basis weights of them can produce absorbent structures having different absorption properties.

As the nonwoven fabrics favorably used in the present invention, there can be used those which include constituent materials such as natural fibers, synthetic fibers, wood pulp, and foams, and are, for example, obtained by such as spun-bond method, melt-blown method, air-through method, spun-lace method, needle-punch method, flocking method, and dry pulp method, and favorably have a basis weight of 10 to 100 g/m$^2$. These base materials may be used in combinations with each other.

As to the fixation base material used in the present invention, such as thickness, density, and opening ratio of it are appropriately selected in such a manner that the average space ratio and average space radius of the absorbent structure can be within the ranges favorable in the present invention in consideration of the previously mentioned average space ratio and average space radius as to spaces between particles of the water-absorbent resin during saturation-swelling without load or under a load of 2.07 kPa (0.3 psi).

For example, in the case where the average space ratio or average space radius of the water-absorbent resin during swelling deviates from the range according to the present invention, sometimes the spaces in the absorbent structure become controlled by appropriately selecting the fixation base material and the hot-melt adhesive for the fixation, so that there is obtained an absorbent structure having the average space ratio and average space radius according to the present invention, and sometimes it is the other way around. Therefore, caution is needed. Specifically, in the case where an attempt is made to maintain the average space ratio and average space radius as intrinsically possessed by the water-absorbent resin, it is enough that a base material having only a small amount of spaces and a thin thickness, therefore, so-called low gas permeability is used as the fixation base material. However, in the case where a base material having a large average space ratio and a large average space radius, therefore, so-called high gas permeability is used, sometimes, the average space ratio and average space radius of the original water-absorbent resin during swelling cannot be maintained, but the average space ratio or average space radius of the resultant absorbent structure is very large. Therefore, caution is needed. In addition, even in the case where the average space ratio or average space radius of the water-absorbent resin during swelling is very small in the other way around deviating from the range according to the present invention, an absorbent structure having the average space ratio and average space radius in the optimum ranges according to the present invention is sometimes obtained by using an optimum fixation base material.

(5) Water-absorbent resin:

The water-absorbent resin, used in the present invention, is a water-insoluble, water-swellable, and hydrogel-formable polymer obtainable by polymerizing a hydrophilic monomer, or is a mixture obtained by adding an additive to this water-swellable polymer. This resin encompasses a mode in which the amount of the additive is smaller than 10 mass % relative to the total of the water-swellable, water-insoluble, and hydrogel-formable polymer and the additive, and this resin has the specific average space ratio and average space radius during swelling with the physiological saline.

Specific examples of the water-insoluble, water-swellable, and hydrogel-formable polymer include: partially-neutralized and crosslinked poly(acrylic acid) polymers (e.g. USP 4,625,001, USP 4,654,039, USP 5,250,640, USP 5,275,773, EP 456136); crosslinked and partially-neutralized graft polymers of starch-acrylic acid (USP 4,076,663); copolymers of isobutylene-maleic acid (USP 4,389,513); saponified copolymers of vinyl acetate-acrylic acid (USP 4,124,748); hydrolyzed (co)polymers of acrylamide (USP 3,959,569); and hydrolyzed polymers of acrylonitrile (USP 3,935,099). Favorable above all are crosslinked polymers comprising poly(acrylic acid) (and/or its salts) wherein the major components of such crosslinked polymers are such as acrylic acid or its salts. As to the crosslinked polymers comprising poly(acrylic acid) (and/or its salts), it is favorable that 50 to 90 mol % of acid groups therein are neutralized, and the salts can be exemplified by such as alkaline metal salts, ammonium salts, and amine salts.

The crosslinked polymers comprising poly(acrylic acid) (and/or its salts), which are water-absorbent resins favorably used in the present invention, may be those which are obtained by, if necessary, copolymerizing another monomer jointly with the monomer used as the major component (for example, the above acrylic acid or its salt). Specific examples of the above other monomer include: anionic unsaturated monomers (e.g. methacrylic acid, maleic acid, vinylsulfonic acid, styrenesulfonic acid, 2-(meth)acrylamido-2-methylpropanesulfonic acid, 2-(meth)acryloylethanesulfonic acid, 2-(meth)acryloylpropanesulfonic acid) and their salts; nonionic hydrophilic-group-containing unsaturated monomers (e.g. acrylamide, methacrylamide, N-ethyl(meth)acrylamide, N-n-propyl(meth)acrylamide, N-isopropyl(meth)acrylamide, N,N-dimethyl(meth)acrylamide, 2-hydroxyethyl (meth)acrylate, 2-hydroxypropyl (meth)acrylate, methoxypolyethylene glycol (meth)acrylate, polyethylene glycol mono(meth)acrylate, vinylpyridine, N-vinylpyrrolidone, N-acryloylpiperidine, and N-acryloylpyrrolidine); and cationic unsaturated monomers (e.g. N,N-dimethylaminoethyl (meth)acrylate, N,N-diethylaminoethyl (meth)acrylate, N,N-dimethylaminopropyl (meth)acrylate, N,N-dimethylaminopropyl(meth)acrylamide, and their quaternary salts). The amount of these monomers other than acrylic acid, as used, is usually in the range of 0 to 30 mol %, favorably 0 to 10 mol %, of the total of all monomers.

As to the water-absorbent resin used in the present invention, the method for introducing a crosslinked structure into it can be exemplified by such as: a method which involves the use of a type that self-crosslinks without any crosslinking agent; and a method which involves copolymerization or reaction with an internal-crosslinking agent having at least two polymerizable unsaturated groups or at least two reactive groups. Favorable is the method which involves the copolymerization or reaction with the internal-crosslinking agent.

Specific examples of these internal-crosslinking agents include N,N'-methylenebis(meth)acrylamide, (poly)ethylene glycol di(meth)acrylate, (poly)propylene glycol di(meth)acrylate, trimethylolpropane tri(meth)acrylate, trimethylolpropane di(meth)acrylate, glycerol tri(meth)acrylate, glycerol acrylate methacrylate, ethylene-oxide-modified trimethylolpropane tri(meth)acrylate, pentaerythritol tetra(meth)acrylate, dipentaerythritol hexa(meth)acrylate, triallyl cyanurate, triallyl isocyanurate, triallyl phosphate, triallylamine, poly(meth)allyloxyalkanes, (poly)ethylene glycol diglycidyl ether, glycerol diglycidyl ether, ethylene glycol, polyethylene glycol, propylene glycol, glycerol, pentaerythritol, ethylenediamine, polyethylenimine, and glycidyl (meth)acrylate. In addition, these internal-crosslinking agents may be used in combinations with each other. From the viewpoint of such as water absorption properties of the resultant water-absorbent polymer, it is favorable that, above all, the compounds having at least two polymerizable unsaturated groups are used essentially as the internal-crosslinking agents, and that the amount thereof as used is in the range of 0.005 to 3 mol %, more favorably 0.01 to 1.5 mol %, relative to the aforementioned monomer components.

Incidentally, when the polymerization is carried out, there may be added the following: hydrophilic polymers such as starch, cellulose, derivatives from starch, derivatives from cellulose, poly(vinyl alcohol), poly(acrylic acid) (and/or its salts), and crosslinked poly(acrylic acid) (and/or its salts); and chain transfer agents such as hypophosphorous acid (and/or its salts).

When the above monomer including acrylic acid or its salt as the major component is polymerized to obtain the water-absorbent resin used in the present invention, bulk polymerization or precipitation polymerization may be carried out, but, from the viewpoint of the performance or the easiness in controlling the polymerization, it is favorable to carry out aqueous solution polymerization or reversed-phase suspension polymerization in which the monomer is used in the form of an aqueous solution. Such polymerization methods have hitherto been known in public and are disclosed in such as USP 4,625,001, USP 4,769,427, USP 4,873,299, USP 4,093,776, USP 4,367,323, USP 4,446,261, USP 4,683,274, USP 4,690,996, USP 4,721,647, USP 4,738,867, and USP 4,748,076.

In addition, when the polymerization is carried out, there may, for example, be used the following: radical polymerization initiators such as potassium persulfate, ammonium persulfate, sodium persulfate, t-butyl hydroperoxide, hydrogen peroxide, and 2,2'-azobis(2-amidinopropane) dihydrochloride; and active energy rays such as ultraviolet rays and electron beams. In addition, in the case where the oxidizable radical polymerization initiators are used, they may be used jointly with reducing agents such as sodium sulfite, sodium hydrogensulfite, ferrous sulfate, and L-ascorbic acid to carry out redox polymerization. The amount of these polymerization initiators as used is usually in the range of 0.001 to 2 mol %, favorably 0.01 to 0.5 mol %.

The shape of the water-absorbent resin, obtained by the above polymerization, is generally such as irregular pulverized shape, spherical shape, fibrous shape, bar shape, approximately spherical shape, or flat shape.

For obtaining the absorbent structure having the specific average space ratio and average space radius according to the present invention, it is favorable that surfaces and their neighborhood of the above water-absorbent resin are further crosslinked with a surface-crosslinking agent. In the case where the surface-crosslinking is not done, sometimes the average space ratio of a saturation-swollen gel (during saturation-swelling) is small, and sometimes it is difficult to obtain the absorbent structure displaying the average space ratio and average space radius in the ranges according to the present invention under a load.

Examples of the surface-crosslinking agent usable for the surface-crosslinking treatment include: polyhydric alcohol compounds (e.g. ethylene glycol, diethylene glycol, propylene glycol, triethylene glycol, tetraethylene glycol, polyethylene glycol, 1,3-propanediol, dipropylene glycol, 2,2,4-trimethyl-1,3-pentanediol, polypropylene glycol, glycerol, polyglycerol, 2-butene-1,4-diol, 1,3-butanediol, 1,4-butanediol, 1,5-pentanediol, 1,6-hexanediol, 1,2-cyclohexanedimethanol, 1,2-cyclohexanol, trimethylolpropane, diethanolamine, triethanolamine, polyoxypropylene, oxyethylene-oxypropylene block copolymers, pentaerythritol, and sorbitol); epoxy compounds (e.g. ethylene glycol diglycidyl ether, polyethylene diglycidyl ether, glycerol polyglycidyl ether, diglycerol polyglycidyl ether, polyglycerol polyglycidyl ether, propylene glycol diglycidyl ether, polypropylene glycol diglycidyl ether, and glycidol); polyamine compounds (e.g. ethylenediamine, diethylenetriamine, triethylenetetramine, tetraethylenepentamine, pentaethylenehexamine, and polyethylenimine) and their inorganic or organic salts (e.g. azetidinium salts); polyisocyanate compounds (e.g. 2,4-tolylene diisocyanate and hexamethylene diisocyanate); polyoxazoline compounds (e.g. 1,2-ethylenebisoxazoline); alkylene carbonate compounds (e.g. 1,3-dioxolan-2-one, 4-methyl-1,3-dioxolan-2-one, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, and 1,3-dioxopan-2-one); haloepoxy compounds (e.g. epichlorohydrin, epibromohydrin, and $\alpha$-methylepichlorohydrin) and their polyamine-added products (e.g. Kymene (registered trademark) produced by Hercules); silane coupling agents (e.g. $\gamma$-glycidoxypropyltrimethoxysilane and $\gamma$-aminopropyltriethoxysilane); and polyvalent metallic compounds (e.g. hydroxides and chlorides of such as zinc, calcium, magnesium, aluminum, iron and zirconium).

The amount of the surface-crosslinking agent, as used, is in the range of about 0.001 to about 5 mass parts, per 100 mass parts of the water-absorbent resin.

In the present invention, when the surface-crosslinking agent and the water-absorbent resin are mixed together, water may be used. The amount of water, as used, is also generally favorably larger than 0.5 but not larger than 10 mass parts, more favorably in the range of 1 to 5 mass parts, per 100 mass parts of the solid content of the water-absorbent resin.

In addition, when the surface-crosslinking agent and/or its aqueous solution is mixed, a hydrophilic organic solvent and/or a third substance may be used. In the case where the hydrophilic organic solvent is used, its examples include: lower alcohols (e.g. methyl alcohol, ethyl alcohol, n-propyl alcohol, isopropyl alcohol, n-butyl alcohol, isobutyl alcohol, and t-butyl alcohol); ketones (e.g. acetone); ethers (e.g. dioxane, tetrahydrofuran, and methoxy(poly)ethylene glycol); amides (e.g. ε-caprolactam and N,N-dimethylformamide); sulfoxides (e.g. dimethyl sulfoxide); and polyhydric alcohols (e.g. ethylene glycol, diethylene glycol, propylene glycol, triethylene glycol, tetraethylene glycol, polyethylene glycol, 1,3-propanediol, dipropylene glycol, 2,2,4-trimethyl-1,3-pentanediol, polypropylene glycol, glycerol, polyglycerol, 2-butene-1,4-diol, 1,3-butanediol, 1,4-butanediol, 1,5-pentanediol, 1,6-hexanediol, 1,2-cyclohexanedimethanol, 1,2-cyclohexanol, trimethylolpropane, diethanolamine, triethanolamine, polyoxypropylene, oxyethylene-oxypropylene block copolymers, pentaerythritol, and sorbitol). Though depending on such as kind, particle diameters, and water content of the water-absorbent resin, the amount of the hydrophilic organic solvent as used is favorably not larger than 50 mass parts, more favorably in the range of 0.1 to 10 mass parts, per 100 mass parts of the solid content of the water-absorbent resin. In addition, as the third substance, there may be caused to coexist those which are disclosed in EP 0668080, such as inorganic acids, organic acids, and polyamino acids.

Although not especially limited, the method for mixing the water-absorbent resin and the surface-crosslinking agent together can be exemplified by such as: a method in which the water-absorbent resin is immersed into the hydrophilic organic solvent and then mixed with the surface-crosslinking agent that is, if necessary, dissolved in water and/or a hydrophilic organic solvent; and a method in which the surface-crosslinking agent that is dissolved in water and/or the hydrophilic organic solvent is spraywise or dropwise added directly to the water-absorbent resin, and then they are mixed together.

After the mixing of the water-absorbent resin and the surface-crosslinking agent, usually, a heating treatment is carried out to conduct the crosslinking reaction. Though depending on the surface-crosslinking agent as used, the temperature of the above heating treatment is favorably in the range of 40 to 250 °C. In the case where the treatment temperature is lower than 40 °C, the absorption properties such as absorption capacity under a load are sometimes not sufficiently be improved. In the case where the treatment temperature is higher than 250 °C, the deterioration of the water-absorbent resin is sometimes caused, so that the performance is lowered, therefore caution is needed. The duration of the heating treatment is in the range of about 1 minute to about 2 hours, favorably about 5 minutes to about 1 hour.

Incidentally, the water-absorbent resin according to the present invention may possess such functions as given or enhanced by causing the water-swellable, water-insoluble, and hydrogel-formable polymer to further contain additives such as: water-insoluble finely-particulate inorganic powders (e.g. silicon dioxide, titanium dioxide, aluminum oxide, magnesium oxide, zinc oxide, talc, calcium phosphate, barium phosphate, silicic acid or its salts, clay, diatomite, zeolite, bentonite, kaolin, hydrotalcite, and salts (e.g. activated clay)); deodorants, perfumes, antibacterial agents, cationic polymer compounds (e.g. polyamines), foaming agents, pigments, dyes, manures, oxidizing agents, reducing agents, chelating agents, and water. The ratio of the additives as used is less than 10 mass %, favorably less than 5 mass %, more favorably less than 1 mass %, relative to the total of the water-swellable crosslinked polymer and the additives.

The water-absorbent resin, used in the present invention production process for the absorbent structure, is favorably ① a water-absorbent resin which displays an average space ratio of 30 to 70 % and an average space radius of 100 to 300 μm as to spaces between particles during saturation-swelling without load or ② a water-absorbent resin which displays an average space ratio of 20 to 50 % and an average space radius of 50 to 130 μm as to spaces between particles during saturation-swelling under a load of 2.07 kPa (0.3 psi).

The average space ratio and average space radius of a saturation-swollen gel of the water-absorbent resin (during saturation-swelling) without load or under the load in the present invention are factors which determine the ability of a water-absorbent resin gel (swollen by water absorption) to further absorb a liquid as thereafter added to the absorbent structure, in the case where water-absorbent resin particles exist in a high concentration to such an extent that they can be adjacent to each other in the absorbent structure. If the average space ratio and average space radius are in the above ranges, the added liquid is sucked by the capillary force of openings formed between particles of the swollen gel, so that the added liquid can reside temporarily between the gel particles. Therefore, even a thin type absorbent structure, from which bulky fibers (e.g. pulp) used for absorbent structures of conventional diapers have been excluded or lessened, can be a leakage-free absorbent structure which rapidly absorbs excreted urine repeatedly without causing the gel-blocking.

In the case where a water-absorbent resin which displays an average space ratio of more than 70 % as to spaces between particles during saturation-swelling without load is used for the production of an absorbent structure according to the present invention, the fixation of the resin to the base material results in that the average space ratio of the absorbent structure is too large, and the sucking-up property of the absorbent structure is deteriorated to such an extent that there is a tendency toward the increase of such as wet-back quantity without good absorption of liquids by the absorbent structure. In the case where a water-absorbent resin which displays an average space ratio of less than 30 % as to spaces between particles during saturation-swelling without load is used, the fixation results in that liquids cannot well permeate the absorbent structure, therefore the absorption rate is so slow that there is a possibility that the leakage may occur from the initial stage. A more favorable water-absorbent resin displays an average space ratio of 45 to 70 % as to spaces between particles during saturation-swelling without load.

In addition, in the case where a water-absorbent resin which displays an average space radius of greater than 300 µm as to spaces between particles during saturation-swelling without load is used for the production of an absorbent structure according to the present invention, the fixation of the resin to the base material results in that the sucking-up property is deteriorated to such an extent that, when the absorbent structure is further used for absorbent articles such as disposable diapers, its ability to suck liquids from other liquid-acquiring base materials such as pulp is deteriorated so greatly as to have a feel of much wetness. In the case where a water-absorbent resin which displays an average space radius of smaller than 100 µm as to spaces between particles during saturation-swelling without load is used, the fixation results in that liquids cannot well permeate the absorbent structure, therefore the absorption rate is so slow that there is a possibility that, when the absorbent structure is used for absorbent articles such as disposable diapers, the leakage may occur from the initial stage. A more favorable water-absorbent resin displays an average space radius of 100 to 250 µm, still more favorably 100 to 200 µm, as to spaces between particles during saturation-swelling without load.

In addition, in the case where a water-absorbent resin which displays an average space ratio of more than 50 % as to spaces between particles during saturation-swelling under a load of 2.07 kPa (0.3 psi) is used for the production of another absorbent structure according to the present invention, the fixation of the resin to the base material results in that the average space ratio of the absorbent structure tends to be too large, and the sucking-up property of the absorbent structure is deteriorated to such an extent that there is a tendency toward the increase of such as wet-back quantity without good absorption of liquids by the absorbent structure under the load. In the case where a water-absorbent resin which displays an average space ratio of less than 20 % as to spaces between particles during saturation-swelling under a load of 2.07 kPa (0.3 psi) is used, similarly, liquids cannot well permeate the absorbent structure under the load, therefore the absorption rate is so slow that there is a possibility that the leakage may occur from the initial stage. A more favorable water-absorbent resin displays an average space ratio of 20 to 50 % as to spaces between particles during saturation-swelling under a load of 2.07 kPa (0.3 psi).

In addition, also as to the case where a water-absorbent resin which displays an average space radius of greater than 130 µm as to spaces between particles during saturation-swelling under a load of 2.07 kPa (0.3 psi) is used for the production of another absorbent structure according to the present invention, similarly, the sucking-up property is deteriorated under the load to such an extent that, when the absorbent structure is further used for absorbent articles such as disposable diapers, its ability to suck liquids from other members such as pulp is deteriorated so greatly that there is a concern about having a feel of much wetness under the load. In the case where a water-absorbent resin which displays an average space radius of smaller than 50 µm as to spaces between particles during saturation-swelling under a load of 2.07 kPa (0.3 psi) is used, liquids cannot well permeate the absorbent structure under the load, therefore the absorption rate is so slow that there is a possibility that the leakage may occur from the initial stage. A more favorable water-absorbent resin displays an average space radius of 50 to 85 µm as to spaces between particles during saturation-swelling under a load of 2.07 kPa (0.3 psi).

It is enough that the water-absorbent resin, used in the present invention, satisfies the above requirement ① or ② , but it is more favorable that both the above requirements ① and ② satisfy the ranges according to the present invention. In the case where only one of these requirements is satisfied, there is a possibility that, depending on conditions for the use of the base material used in the case of fixing the water-absorbent resin, the absorption properties of the absorbent structure may not well be displayed.

For obtaining an absorbent structure according to the present invention which involves the fixation with the hot-melt adhesive and displays a capillary absorption capacity of not less than 10 (g/g) at a height of 20 cm, it is favorable to use a water-absorbent resin which also displays a capillary absorption capacity of not less than 10 (g/g) at a height of 20 cm. Even if a water-absorbent resin which displays a capillary absorption capacity of less than 10 (g/g) at a height of 20 cm is used, an absorbent structure which displays a capillary absorption capacity of not less than 10 (g/g) at a height of 20 cm is sometimes obtained dependently on the kind of the base material and the method for the fixation. However, the capillary absorption capacity generally tends to be deteriorated by combination with the base material, therefore caution is needed.

Therefore, it is favorable to use a water-absorbent resin which displays a capillary absorption capacity of not less than 15 (g/g) at a height of 20 cm, more favorably a water-absorbent resin which displays a capillary absorption capacity of not less than 20 (g/g) at a height of 20 cm, still more favorably a water-absorbent resin which displays a capillary absorption capacity of not less than 25 (g/g) at a height of 20 cm.

These ones are obtained by optimizing such as: the average particle diameter and particle diameter distribution of the above water-absorbent resin; the surface-crosslinking agent; and the thickness of a surface-crosslinked layer. For example, its method can be exemplified by processes such as ① to ③ below.

① A process including the step of carrying out a surface-crosslinking treatment so that the resultant surface-crosslinked layer can be in the range of 200 to 600 nm by heat-treating a carboxyl-group-containing irregular pulverized water-absorbent resin, having an average particle diameter of 300 to 500 µm, in the presence of a polyhydric alcohol, or an alkylene carbonate, or an oxazolidone compound.

② A process including the steps of: carrying out a surface-crosslinking treatment so that the resultant surface-crosslinked layer can have a thickness of 200 to 600 nm by heat-treating a carboxyl-group-containing water-absorbent resin, having an average particle diameter of 100 to 500 μm, more favorably 200 to 400 μm, in the presence of a surface-crosslinking agent reactable with the carboxyl group; and then adding water-insoluble fine particles.

③ A process including the steps of: heat-treating a carboxyl-group-containing water-absorbent resin, having an average particle diameter of 100 to 300 μm, more favorably 200 to 250 μm, in the presence of a surface-crosslinking agent reactable with the carboxyl group; and then adding a cationic polymer having molecular weights in a specific range.

Incidentally, how to determine the thickness of the surface-crosslinked layer is disclosed in such as the specification of Japanese Patent Application No. 2000-329501. The cationic polymer is exemplified in such as JP-A-031360/1993 (Kokai) and JP-A-000370/1994 (Kokai).

The water-absorbent resins obtained by such methods are favorably usable.

(6) Hot-melt adhesive:

The hot-melt adhesive, used in the present invention, is a material which, under heated conditions, melt-fluidizes to, thus in various shapes, be added to another material and, at normal temperature, solidifies to thus enable the adhesion and fixation of this material, and includes a synthetic resin as the major component and, further in addition thereto if necessary, includes an additive such as tackifier and softening agent. Examples of the synthetic resin, used for the hot-melt adhesive, include rubbers (e.g. natural rubber, butyl rubber, polyisoprene rubber), styrenic resins (e. g. SBS, SIS, SEBS, SEPS), ethylenic copolymers (e.g. EVA), polyolefin resins, polyester resins, polyamide resins, acrylic resins, and polyurethane resins.

Above all usable favorably for the present invention are such as: ethylenic copolymers (e.g. ethylene-vinyl acetate copolymers, ethylene-acrylate ester copolymers (EVA), ethylene-methyl acrylate copolymers (EMA), ethylene-nbutyl acrylate copolymers (EnBA)); polyolefins (e.g. atactic polypropylene (APP), low-density polyethylene (LDPE), amorphous polyalphaolefin (APAO)); styrenic elastomers (e.g. styrene-butadiene-styrene copolymers (SBS), styrene-isoprene-styrene copolymers (SIS), styrene-ethylene-butylene-styrene copolymers (SEBS), styrene-ethylene-propylene-styrene copolymers (SEPS)); and block copolymers of acrylate ester-methacrylate ester-styrenic monomer.

Furthermore, the above synthetic resins may be mixed with tackifying resins (e.g. rosins, rosin derivatives, polyterpene resins, polyterpene resin derivatives, aliphatic hydrocarbon resins and hydrogenated products of this type of resins, alicyclic hydrocarbon resins and hydrogenated products of this type of resins, aromatic hydrocarbon resins and hydrogenated products of this type of resins), or softening agents (e.g. naphthenic process oils, paraffinic process oils, animal and plant oils and their derivatives, polyolefinic low-molecular polymers).

As the method for supplying the above hot-melt adhesive onto the water-absorbent resin layer, there are a contact type and a non-contact type, but the non-contact type is favorable in view of such as: heat resistance and unevenness of the base material which receives the supply; influence on the water-absorbent resin; and amount of the supply. Examples of the non-contact type include: melt-blow methods (curtain-spraying method and slot-spraying method) which enables comparatively dense coating and dispersing: a spiral-spraying method which is not so dense as the melt-blow methods; or a method including the steps of dispersing or coating the hot-melt adhesive onto a release sheet and then transferring the hot-melt adhesive onto the base material or the water-absorbent resin placed thereon. Any of them is usable for the present invention.

As to apparatus for supplying the hot-melt adhesive, examples of the apparatus for the melt-blow method include: Dynafiber "UFD" Coating Apparatus produced by ITW Dynatec; and Control Coat Gun Coating Apparatus produced by Nordson. Examples of the apparatus for the spiral-spraying method include: Swall Coating Gun Apparatus produced by ITW Dynatec; and Spiral Spray Gun Coating Apparatus produced by Nordson. Favorably, the melt-blow method is adopted.

In the present invention, favorably, the hot-melt adhesive is supplied in the shape of fine fibers and, more favorably, the fine fibers are net-cast onto the water-absorbent resin layer in the shape of a web or dispersed so as to envelop the water-absorbent resin layer. Their average fiber diameter is in the range of 0.1 to 300 μm. In the case where the fiber diameter is greater than 300 μm, the texture of the absorbent structure is deteriorated, and further, restrictions are imposed on the swelling of the water-absorbent resin, and also, a large amount of hot-melt adhesive is needed for the fixation. In addition, in the case where the fiber diameter is smaller than 0.1 μm, there is a possibility that the strength of the fibers may be so insufficient as to be unable to fix the water-absorbent resin enough. The average fiber diameter is favorably in the range of about 5 to about 100 μm, more favorably about 5 to about 50 μm. In addition, hereupon, the average diameter as to intervals between fibers of the web net has relations also with such as average particle diameter of the water-absorbent resin as used, but, usually, is set at not greater than the average particle diameter possessed by the water-absorbent resin during its swelling, and it is favorable to carry out the dispersion so that the average diameter as to intervals between fibers of the web net can be in the range of 10 to 1,000 µm. In the case where the average diameter as to intervals between fibers of the web net is smaller than 10 µm, the absorption rate of the water-absorbent resin is greatly decelerated, or the swelling is greatly restricted. In addition, in the case where the average diameter as to intervals between fibers of the web net is greater than 1,000 µm, the web interval is larger than particles of the water-absorbent resin during its swelling, so that the water-absorbent resin is not fixed enough. The average diameter as to intervals between fibers of the web net is favorably in the range of 10 to 500 µm.

The amount of the hot-melt adhesive as supplied is usually in the range of 0.1 to 50 $g/m^2$. In the case where this amount is smaller than 0.1 $g/m^2$, the resultant net is sparse, thin, and weak, and it is therefore impossible to obtain sufficient adhesive strength. In the case where the above amount is larger than 50 $g/m^2$, the fiber diameter is so great as to be filmy, resulting in inhibiting the water absorption functions. The above amount is favorably in the range of 0.2 to 20 $g/m^2$.

The hot-melt adhesive, used in the present invention, is supplied in the shape of fine fibers and, more favorably, the fine fibers are net-cast onto a surface layer of the water-absorbent resin in the shape of a web, or dispersed so as to envelop the water-absorbent resin, or mixed with water-absorbent resin particles. Therefore, in the case where the hot-melt adhesive is sprayed by the above melt-blow-spraying method in order to achieve this object, the melt viscosity of the hot-melt adhesive at 164°C is favorably in the range of about 700 to about 40,000 mPa·s, more favorably about 2,000 to about 7,000 mPa·s. In the case where the melt viscosity of the hot-melt adhesive at 160 °C is not more than 700 mPa·s, sufficient adhesive strength is sometimes not obtained. In the case where the melt viscosity of the hot-melt adhesive at 160 °C is more than 40,000 mPa·s, there is a possibility that it may be impossible to carry out uniform dispersion.

In addition, in the case where the hot-melt adhesive, used in the present invention, has a purpose of, even in a dry state, sufficiently fixing the water-absorbent resin in the absorbent structure, it is favorable to select a hot-melt adhesive of the composition having an appropriate tack property. Though much depending on the amount of the hot-melt adhesive as supplied, if the tack property is high, there sometimes occur problems such that fibers of the hot-melt adhesive attach to another material in the case of using the absorbent structure for absorbent articles. However, as mentioned below, the use of the above property enables the production of an absorbent structure which is unprecedentedly featured in that the degree of the fixation varies with the increase of the amount of a liquid absorbed by the absorbent structure. In addition, in the case where the attachment is problematic, this attachment can be avoided by only causing an anti-sticking agent (which is typified by water-absorbent resins as also mentioned below) to exist in a small amount between the material possible for the hot-melt adhesive fibers to attach to and the hot-melt adhesive fibers.

As to the hot-melt adhesive in the present invention, in the case where a paper base material is caused to adhere onto fine fibers of the hot-melt adhesive of 10 $g/m^2$ as coated onto the water-absorbent resin layer of 360 $g/m^2$, it is sometimes favorable to use a hot-melt adhesive of not less than 20 % in ratio of migration to the paper base material, though depending on the above-illustrated concept of designing the absorbent structure. The ratio of migration is indicated by a ratio of an area of the hot-melt adhesive, migrating to the paper base material when the paper base material is lifted from the absorbent structure after being put thereon and press-attached thereto, to an area of the absorbent structure as originally coated with the hot-melt adhesive in the shape of a web of its fine fibers. In the case where the ratio of migration is less than 20 %, the pressure sensitive adhesive property of the water-absorbent resin in a dry state is sometimes inferior, so it is sometimes impossible to produce the above absorbent structure having a water-absorbent resin layer of which the degree of fixation varies. In the case where the absorbent structure having a water-absorbent resin layer of which the degree of fixation varies is produced, the ratio of migration of the hot-melt adhesive to the paper base material is favorably in the range of 50 to 100 %.

Furthermore, as to the hot-melt adhesive used in the present invention, it has an elongation of not less than 100 % at 25 °C favorably for being able to follow the swelling of the water-absorbent resin. Hereupon, the elongation can be indicated as a ratio of the height of a cone to the diameter of the bottom of the cone as to a conical net as formed when a portion of the hot-melt adhesive is picked up with tweezers and then lifted vertically into a conical shape after the hot-melt adhesive has been supplied onto release paper in the shape of a web of fine fibers. In the case where the elongation is less than 100 %, the elongation of the fibers does not accompany the swelling of the water-absorbent resin, therefore the restrictions on the swelling tend to occur, and the degree of the fixation is also weak. The elongation at 25 °C is favorably not less than 200 %, more favorably not less than 300 %, still more favorably not less than 500 %.

(7) Production process for absorbent structure:

In the present invention production process for an absorbent structure, the water-absorbent resin is supplied onto the base material by methods such as dispersion. In the case where another material is if necessary added to the water-absorbent resin, the above supply is carried out by a method, for example, in which: the water-absorbent resin and another material are premixed together and then the resultant mixture is dispersed, or the water-absorbent resin and another material are separately dispersed onto the base material (the timing of their dispersion may be the same time or different times); and then the resultant water-absorbent resin layer is fixed to the base material with such as hot-melt adhesive. As means for the fixation to the base material, there is preferred a method involving the use of the aforementioned hot-melt adhesive.

For preventing the water-absorbent resin layer from flying in all directions or falling off during the production of the absorbent structure, it is favorable to temporarily fix the water-absorbent resin layer to the base material with a temporarily fixing agent before supplying the hot-melt adhesive. As the temporarily fixing agent, there are preferred those which themselves have the pressure sensitive adhesive property or those which tackify the water-absorbent resin by contacting with the water-absorbent resin layer. Specific examples thereof include: water; lower alcohols (e. g. methyl alcohol, ethyl alcohol, n-propyl alcohol, isopropyl alcohol, n-butyl alcohol, isobutyl alcohol, and t-butyl alcohol) and their aqueous solutions; polyhydric alcohol compounds (e.g. ethylene glycol, diethylene glycol, propylene glycol, triethylene glycol, tetraethylene glycol, polyethylene glycol, 1,3-propanediol, dipropylene glycol, 2,2,4-trimethyl-1,3-pentanediol, polypropylene glycol, glycerol, polyglycerol, 2-butene-1,4-diol, 1,3-butanediol, 1,4-butanediol, 1,5-pentanediol, 1,6-hexanediol, 1,2-cyclohexanedimethanol, 1,2-cyclohexanol, trimethylolpropane, diethanolamine, triethanolamine, polyoxypropylene, oxyethylene-oxypropylene block copolymers, pentaerythritol, and sorbitol) and their aqueous solutions; ketones (e.g. acetone) and their aqueous solutions; cationic compounds (e.g. polyamines) and their aqueous solutions; and solution adhesives, emulsion adhesives, and pressure sensitive adhesives, wherein these adhesives include rubbers and thermoplastic resins as major components. Above all, the use of water as the temporarily fixing agent is favorable from the viewpoint of safety and prices.

The temporarily fixing agent may be supplied in any stage of before and/or after supplying the water-absorbent resin layer to the base material.

The method for supplying the temporarily fixing agent is not especially limited. However, a spraying method involving the use of a spray gun is favorable for obtaining the temporarily fixing effect sufficiently by use of a small amount.

The amount of the temporarily fixing agent as used is favorably in the range of 0.1 to 30 mass parts, more favorably 5 to 20 mass parts, per 100 mass parts of the water-absorbent resin layer. In the case where the amount of the temporarily fixing agent is smaller than 0.1 mass part, the temporary fixation of the water-absorbent resin layer is sometimes so insufficient that the water-absorbent resin layer flies in all directions or falls off during the supply of the hot-melt adhesive. In addition, in the case where the amount of the temporarily fixing agent is larger than 30 mass parts, the affinity between the water-absorbent resin layer and the hot-melt adhesive is sometimes so bad that the fixability of the water-absorbent resin layer is bad.

The amount of the hot-melt adhesive as supplied is usually in the range of 0.1 to 50 g/m$^2$. In the case where this amount is smaller than 0.1 g/m$^2$, the resultant net is sparse, thin, and weak, and it is therefore impossible to obtain sufficient adhesive strength. In the case where the above amount is larger than 50 g/m$^2$, the fiber diameter is so great as to be filmy, resulting in inhibiting the water absorption functions. The above amount is favorably in the range of 0.2 to 20 g/m$^2$.

The amounts of the base material, the water-absorbent resin layer, and the hot-melt adhesive, as used during the production of the absorbent structure, is as follows: per 100 mass parts of the water-absorbent resin layer, the base material is in the range of about 0.1 to about 50 mass parts, and the hot-melt adhesive is in the range of about 0.001 to about 10 mass parts, and favorably, the base material is in the range of about 1 to about 20 mass parts, and the hot-melt adhesive is in the range of about 0.01 to about 1 mass part.

As previously mentioned, the absorbent structure according to the present invention, which involves the fixation with the hot-melt adhesive, displays an average space ratio of 30 to 70 % and an average space radius of 100 to 300 μm during saturation-swelling without load, or displays an average space ratio of 20 to 50 % and an average space radius of 50 to 130 μm during saturation-swelling under a load of 2.07 kPa (0.3 psi).

The absorbent structure, according to the present invention, is obtained by using the above water-absorbent resin which displays the specific average space ratio and the specific average space radius, or by controlling the combination of such as the properties (opening ratio, thickness, density) of the base material, the properties (shape, average particle diameter, particle diameter distribution, water absorption performances) of the water-absorbent resin, and the method for adhesion between the hot-melt adhesive and the water-absorbent resin layer.

The pattern (method) for supplying the water-absorbent resin and the hot-melt adhesive can be exemplified by such as the following processes ① to ⑥ .

① A process including the steps of supplying the water-absorbent resin layer onto the base material uniformly so as to form a single layer, and then supplying the hot-melt adhesive onto the resin layer and the base material, thereby coating them.

② A process including the steps of supplying the water-absorbent resin layer onto the base material non-uniformly or intermittently (into the shape of such as stripes), and then supplying the hot-melt adhesive onto the resin layer and the base material, thereby coating them.

③ A process including the steps of supplying the water-absorbent resin layer onto the base material patternedly, and then supplying the hot-melt adhesive onto the resin layer and the base material, thereby coating them.

④ A process including the steps of supplying the hot-melt adhesive and the water-absorbent resin layer onto the base material alternately so as to form a multiple layer, thereby fixing them.

⑤ A process including the steps of supplying the water-absorbent resin layer into an opening portion of the base material in a manner for the water-absorbent resin layer to partially be embedded in the opening portion, and then supplying the hot-melt adhesive onto the resin layer and the base material, thereby coating them.

⑥ A process including the steps of supplying the hot-melt adhesive onto the water-absorbent resin layer and a first base material in a state where a portion of the water-absorbent resin layer is passed through an opening portion of the first base material to attain onto a second base material placed beneath the first base material, thereby coating them.

In the case where the supply is carried out so as to form the multiple layer, the kinds of the water-absorbent resin and of the hot-melt adhesive may be the same as or different from each other respectively.

In addition, in the case of ⑥ above, there can be produced a characteristic absorbent structure in which particles of the water-absorbent resin are automatically classified according to the size of the opening portion of the first base material, so that a water-absorbent resin having comparatively coarse particle diameters is placed on the first base material, and that a water-absorbent resin having comparatively coarse particle diameters is placed on the second base material.

As long as a liquid absorption system intended by the present invention is not hindered, another member may further be included in the absorbent structure according to the present invention. Examples of this other member include hydrophilic fibers, nonwoven fabrics, paper, and tissues. Examples of the above hydrophilic fibers include: cellulose fibers obtained from wood (e.g. mechanical pulp, chemical pulp, semichemical pulp, dissolving pulp); and fibers (e.g. rayon, acetate). However, there is no limitation thereto.

In the case where, as previously mentioned, the hot-melt adhesive fibers have the tack property, for, example, have the pressure sensitive adhesive property at room temperature, there is the following phenomenon. In the case where there is produced an absorbent structure involving the fixation done by coating the hot-melt onto the water-absorbent resin layer into the shape of a net of fibers in the pattern such as ① to ⑥ above, if another member (e.g. paper) is laminated onto this absorbent structure, then the hot-melt adhesive fibers sometimes adhere to the paper, so that the fixation of the water-absorbent resin is not sufficiently done.

For avoiding such a phenomenon, it is favorable to cause an anti-sticking material to exist on the hot-melt fiber net. The above anti-sticking agent is usable without especial limitation if it inhibits the attachment between the hot-melt adhesive and another member (e.g. paper) by existing therebetween. However, for efficiently carrying out the fixation of the water-absorbent resin layer without hindering the elongation of the hot-melt adhesive during the liquid absorption, it is favorable to use a hydrophilic release material, having high hydrophilicity, as the above anti-sticking agent. Favorable as the hydrophilic release material are those which have high hydrophilicity (favorably, high water absorbency) and are exemplified by such as water-absorbent resins, hydrophilic inorganic powders, hydrophilic organic powders, and hydrophilic fibers. Above all, the water-absorbent resins are preferable. The anti-sticking material may be supplied onto the hot-melt fiber net after the supply of the hot-melt adhesive or simultaneously with this supply.

In addition, in the case where the hot-melt adhesive fibers have the tack property and thus have the pressure sensitive adhesive property at room temperature, a novel absorbent structure or article such that the fixation ratio of the water-absorbent resin decreases with the passage of time to use the absorbent structure can be produced by supplying the hot-melt adhesive and the water-absorbent resin onto the base material, for example, alternately so as to form a multiple layer, to thereby fix them, and then bringing a member adherent to the hot-melt adhesive fibers (e. g. paper) into contact with the resultant absorbent structure.

In this case, an outermost first hot-melt adhesive fibers, first of all, adhere to the paper, and a first water-absorbent resin layer underlying them becomes gradually mobile by being pressurized after absorbing a liquid, but, under it, there further lies a second water-absorbent resin layer fixed with a second hot-melt adhesive fibers. If the first water-absorbent resin which is an upper layer is moved by external force, the second hot-melt adhesive fibers comes to easily adhere to the paper next, and the second water-absorbent resin layer also becomes easy to mobilize. Thus, if the member which easily adheres to the hot-melt adhesive fibers having a high tack property is combined with the placement of the water-absorbent resin which acts as the above hydrophilic release agent having a low adhesive property, then there is obtained: an absorbent structure such that the fixation ratio of the water-absorbent resin decreases with the passage of time by pressurization; or an absorbent structure such that: some water-absorbent resin layers are fixed, but other water-absorbent resins are not. If such an absorbent structure is used for absorbent articles such as disposable diapers, a portion of the originally fixed water-absorbent resin becomes mobile with the increase of the urination amount, so that there can be given an excellent property of being able to reduce a body-compressed state caused by volume expansion of the water-absorbent resin.

Furthermore, for example, in the case where the hot-melt adhesive and the water-absorbent resin layer are multistepwise supplied, it is also possible that the ratio between a fixed water-absorbent resin and a non-fixed water-absorbent resin is controlled arbitrarily within the range of (about 10 : about 90) to (about 90 : about 10) by more optimizing the kind of the hot-melt adhesive and its supply position, for example, inside fixing the resin with a low-tack hot-melt adhesive and outside fixing the resin with a high-tack hot-melt adhesive.

Thus, dependently on the purpose, it is unnecessary that the water-absorbent resin layers in the absorbent structure are all sufficiently fixed and, if only a part of the water-absorbent resin layers are fixed to the end, then the water-absorbent resin does not completely become distributed unevenly into a portion of the absorbent structure or article during use, therefore there can be avoided the problem of the leakage involved in the migration of the water-absorbent resin, and further, there can also be solved the problem of the pressure as put on bodies by volume expansion of the absorbent structure accompanying the increase of the absorption quantity.

Incidentally, also as to the absorbent structure according to the present invention, it is possible to give the absorbent structure further functions by further adding thereto such as: water-insoluble finely-particulate inorganic powders (e.g. silicon dioxide, titanium dioxide, aluminum oxide, magnesium oxide, zinc oxide, talc, calcium phosphate, barium phosphate, silicic acid or its salts, clay, diatomite, zeolite, bentonite, kaolin, hydrotalcite, and salts (e.g. activated clay)); deodorants, perfumes, antibacterial agents, cationic polymer compounds (e.g. polyamines), foaming agents, pigments, dyes, hydrophilic short fibers, manures, oxidizing agents, reducing agents, chelating agents, and water.

(8) Constitution of absorbent structure (second absorbent structure):

The absorbent structure, according to the present invention, comprises a water-absorbent resin as an essential material and, if necessary, further comprises another material.

Examples of favorable modes for carrying out the absorbent structure according to the present invention include:

a mode that is an absorbent structure which comprises a water-absorbent resin (water-absorbent resin particles) as an essential material;

wherein the water-absorbent resin (water-absorbent resin particles) exists in a ratio of not less than 50 mass % in the absorbent structure, wherein the water-absorbent resin has properties of displaying an average space ratio of 45 to 70 %, favorably 45 to 60 %, and an average space radius of 100 to 200 μm as to spaces between particles during saturation-swelling with a physiological saline (a 0.9 mass % aqueous NaCl solution) without load, and further displaying a capillary absorption capacity of at least 20 g/g; or
wherein the water-absorbent resin (water-absorbent resin particles) exists in a ratio of not less than 50 mass % in the absorbent structure, wherein the water-absorbent resin has properties of being a water-absorbent resin that displays an average space ratio (S1) of 45 to 70 %, favorably 45 to 60 %, and an average space radius (R1) of 100 to 200 μm as to spaces between particles during saturation-swelling with a physiological saline (a 0.9 mass % aqueous NaCl solution) without load, and further displays an average space ratio (S2) of 20 to 50 %, favorably 30 to 40 %, and an average space radius (R2) of 50 to 85 μm, favorably 50 to 75 μm, as to spaces between particles during saturation-swelling with a physiological saline (a 0.9 mass % aqueous NaCl solution) under a load of 2.07 kPa (0.3 psi).

Hereinafter, in the present specification, the absorbent structure as recited in the present item (8) may be referred to as "second absorbent structure". In addition, unless otherwise noted, the statement of the present item (8) is made as a description about the second absorbent structure.

In the absorbent structure according to the present invention, the water-absorbent resin having the above properties is used in such an amount that its content is not less than 50 mass % relative to the entirety of the absorbent structure, for the purpose of making it possible to easily solve the aforementioned problems, but the above content is favorably not less than 70 mass %, more favorably not less than 80 mass %, still more favorably not less than 90 mass %. If the water-absorbent resin having the above properties is contained in the amount of not less than 50 mass % of the absorbent structure, it is, for example, possible to easily obtain an absorbent structure which has a high ratio of the water-absorbent resin as used, and is a thin type, and further has excellent water absorption properties such as high water absorbency. Incidentally, as to the upper limit of the above content, it can be said to favorably be 100 mass % (or substantially 100 mass %) from the viewpoint of obtaining a product containing the water-absorbent resin in a high concentration. However, considering the actual working circumstances such as: stability of the shape of the absorbent structure or the absorbent article comprising it and their handling properties and the environment of their uses (e.g. circumstances where there is a possibility that such as uneven distribution or deformation of the absorbent structure takes place before or during the use of the absorbent article and thus causes the leakage); or various performances and the balancing therebetween of the absorbent structure or the absorbent article comprising it; then there is a possibility that, if the water-absorbent resin content is too high, then, in the other way around, it may not match the above actual working circumstances. In such a case, if it is arranged that a layer of the water-absorbent resin should be contained as the major constituent material and that a layer which can improve such as absorption properties or texture should auxiliarily additionally be contained, then the improvement is sometimes achieved effectively. Hereupon, the upper limit of the above water-absorbent resin content can be considered to favorably be 98 mass % and also can be considered to more favorably be 95 mass % if necessary. Examples of the above layer, which is auxiliarily added, include: the previously mentioned adhesive layer; fibrous material layer such as nonwoven fabric; and porous material layer such as foam.

In the absorbent structure according to the present invention, favorable examples of the above other material, which gets contained if necessary, include those which enhance water absorption performances of the water-absorbent resin layer or give it functions, and specific favorable examples thereof include the same as enumerated in the description of the first absorbent structure.

The amount of the present invention absorbent structure as used for absorbent articles is usually in the range of about 100 to about 2,000 g/m$^2$. In the case where this amount is smaller than 250 g/m$^2$, the saturated absorption quantity of the absorbent structure is so small that there is seen a tendency for the comfortableness to deteriorate (for the feel of dryness to be so inferior that the wet-back quantity increases) when the absorbent structure is used for body-fluid-absorbent articles. The amount of the absorbent structure is favorably in the range of about 200 to about 1,500 g/m$^2$, more favorably about 300 to about 800 g/m$^2$.

In the absorbent structure according to the present invention, the water-absorbent resin having the above specific properties is used, but, to this water-absorbent resin, there can basically be applied the same description as of the water-absorbent resin as used for the first absorbent structure.

Incidentally, as to the case where there is used the water-absorbent resin of which the average space ratio and average space radius as to spaces between particles during saturation-swelling without load do not satisfy the above ranges and as to the case where there is used the water-absorbent resin of which the average space ratio and average space radius as to spaces between particles during saturation-swelling under a load of 2.07 kPa (0.3 psi) do not satisfy the above ranges, there is the same tendency or possibility as described about the first absorbent structure.

The water-absorbent resin having the above specific properties can be obtained by optimizing such as: the average particle diameter and particle diameter distribution of a water-absorbent resin; the surface-crosslinking agent; and the thickness of a surface-crosslinked layer. For example, its method can be exemplified by processes such as ① to ③ below.

① A process including the step of carrying out a surface-crosslinking treatment so that the resultant surface-crosslinked layer can be in the range of 200 to 600 nm by heat-treating a carboxyl-group-containing irregular pulverized water-absorbent resin, having an average particle diameter of 100 to 400 µm, in the presence of a polyhydric alcohol, or an alkylene carbonate, or an oxazolidone compound.

② A process including the steps of: carrying out a surface-crosslinking treatment so that the resultant surface-crosslinked layer can have a thickness of 200 to 600 nm by heat-treating a carboxyl-group-containing water-absorbent resin, having an average particle diameter of 75 to 400µm, more favorably 100 to 300 µm, in the presence of a surface-crosslinking agent reactable with the carboxyl group; and then adding water-insoluble fine particles.

③ A process including the steps of: heat-treating a carboxyl-group-containing water-absorbent resin, having an average particle diameter of 75 to 300 µm, more favorably 100 to 200 µm, in the presence of a surface-crosslinking agent reactable with the carboxyl group; and then adding a cationic polymer having molecular weights in a specific range.

Incidentally, as to how to determine the thickness of the surface-crosslinked layer and as to the cationic polymer, it is enough to refer to such as the documents as cited in the description of the first absorbent structure.

As to the production process for the absorbent structure according to the present invention, production techniques (such as processes and conditions) which have hitherto been known in public for the production of absorbent structures can be applied thereto except to use the above specific water-absorbent resin in the specific ratio. In addition, it is also possible to apply thereto the same production process as that for the first absorbent structure.

(9) Absorbent article:

The absorbent article, according to the present invention, generally comprises the aforementioned (first or second) absorbent structure according to the present invention, a liquid-permeable sheet, and a liquid-impermeable sheet, wherein the absorbent structure is interposed between the liquid-permeable sheet and the liquid-impermeable sheet by adhesion. Then, because of having the above-constituted absorbent structure, this absorbent article has the above excellent water absorption properties. Examples of the absorbent article include: sanitary materials such as disposable diapers, sanitary napkins, and so-called incontinent pads; medical sheets; and condensed-water-absorbing sheets. However, there is no especial limitation thereto.

When the absorbent structure according to the present invention is interposed between the liquid-permeable sheet and the liquid-impermeable sheet, the direction of the absorbent structure is not especially limited. In addition, as to the first absorbent structure, the base material to which the water-absorbent resin layer is fixed may be placed on the liquid-permeable sheet side of the absorbent article and selected within the range of the present invention to thus be given a role in such as acquiring a liquid or diffusing and sucking up a liquid.

The material referred to as the above liquid-permeable sheet is a material having a property of permeating an aqueous liquid, and examples thereof include: nonwoven fabrics; woven fabrics; and porous synthetic resin films including such as polyethylene, polypropylene, polyester, and polyamide. The above liquid-impermeable sheet is a material having a property of not permeating any aqueous liquid, and examples thereof include: synthetic resin films including such as polyethylene, polypropylene, ethylene vinyl acetate, and polyvinyl chloride; films including composite materials of these synthetic resins and nonwoven fabrics; and films including composite materials of the above synthetic resins and woven fabrics. Incidentally, the liquid-impermeable sheet may have a property of permeating a vapor.

Besides the absorbent structure, another member may be used for the absorbent article according to the present invention. This other member can be exemplified by such as a liquid-acquiring member and a liquid-diffusing and liquid-sucking-up member.

The liquid-acquiring member usable in the present invention is a material which is excellent in the abilities to acquire and release a liquid, and the liquid-diffusing and liquid-sucking-up member usable in the present invention is a material which is excellent in the abilities to suck up, transport, and store a liquid. If these are jointly used, there is sometimes obtained an absorbent article which is more excellent in momentary liquid absorption, temporary liquid storage, and liquid absorption efficiency.

Such as the liquid-acquiring member and the liquid-diffusing and liquid-sucking-up member, usable in the present invention, are, as previously mentioned, exemplified by such as: paper, pulp, crosslinked cellulose fibers, synthetic fibers (e.g. nonwoven fabrics), porous polymers (sheets) obtained by polymerization of high internal phase emulsion (HIPE), foams comprising synthetic polymers (e.g. polyurethane, polystyrene, polyethylene, polypropylene, polyester, polyvinyl alcohol, butadiene-styrene rubber (SBR), nitrile-butadiene rubber); fiber aggregates formed by adhesion or biding of synthetic fibers (e.g. polyethylene, polypropylene, polyethylene terephthalate, nylon); rayon fibers; and fiber aggregates formed by press-attaching, adhesion, or biding of hydrophilic fibers (e.g. cellulose fibers (e.g. cellulose, cellulose acetate, nitrocellulose), polyamide fibers). Favorably, the liquid-acquiring member can be exemplified by such as: the pulp; the crosslinked cellulose fibers; the synthetic fibers (e.g. nonwoven fabrics) having bulky structures; and the porous polymers (sheets) obtained by polymerization of high internal phase emulsion (HIPE); and further, the liquid-diffusing and liquid-sucking-up member, usable in the present invention, can be exemplified by such as: the porous polymers (sheets) obtained by polymerization of high internal phase emulsion (HIPE); and high-density cellulose fiber sheets. Also the shape of the liquid-acquiring member and the liquid-diffusing and liquid-sucking-up member can be the shape of such as sheets, fibers, fiber aggregates, particles, and strips, but generally the shape of sheets is favorable. Hereupon, the basis weight of the liquid-acquiring member is in the range of about 50 to about 500 g/m$^2$, favorably about 100 to about 200 g/m$^2$.

As mentioned above, as to the first absorbent structure according to the present invention, because, in the case where the water-absorbent resin layer is fixed to the base material, the hot-melt adhesive is used to control within the specific ranges the average space ratio and average space radius as to spaces between particles of the water-absorbent resin during saturation-swelling or the average space ratio and average space radius of the resultant absorbent structure during saturation-swelling, there is obtained an absorbent structure of which the water-absorbent resin layer is fixed effectively without falling off, and which little becomes deformed when used as an absorbent structure for such as diapers, and which is excellent in the properties of permeating, diffusing, and sucking up a liquid. The use of such an absorbent structure makes it possible to provide a thin type absorbent article which displays very excellent liquid-absorbing ability. Furthermore, there can be produced a thin type absorbent structure and a thin type absorbent article which are, by selecting the specific hot-melt adhesive and its supply method, freed from the restrictions, due to the fixation of the water-absorbent resin, on the swelling, and excellent also in fixability in a dry state before use, and of which the absorbing portion is, by specifying the constitution for supplying the water-absorbent resin and the hot-melt adhesive, prevented from becoming bulky, because the water-absorbent resin as fixed to the base material is released from the fixing force little by little with the progress of the swelling of the water-absorbent resin to thus become mobile.

In addition, as to the second absorbent structure according to the present invention, because the water-absorbent resin, of which the average space ratio and average space radius as to spaces between particles during saturation-swelling are controlled within the specific ranges, is used in the specific ratio, there is obtained an absorbent structure in which the water-absorbent resin is contained in a high concentration, and which is excellent also in such as properties of permeating, diffusing, and sucking up a liquid when used as an absorbent structure for such as diapers. The use of such an absorbent structure makes it possible to provide a thin type absorbent article which displays very excellent liquid-absorbing ability.

The performances of the liquid-acquiring member, the water-absorbent resin, the absorbent structure, and the absorbent article were measured by the following methods.

As the specimen such as water-absorbent resin, there were used those which had been preserved in a moisture-impermeable container such as polypropylene-made airtight container. The following various measurements were carried out under conditions of 25±2 °C and 60±5 % RH.

1. Average space ratio and average space radius of absorbent structure during saturation-swelling and average space ratio and average space radius as to spaces between particles of water-absorbent resin during saturation-swelling:

The average space ratios and average space radii of the absorbent structure and of the water-absorbent resin during saturation-swelling are measured with a measurement apparatus as shown in Fig. 1.

The height h by which a liquid rises by the capillary force through a tube having a radius R is indicated by $h = 2\gamma\cos\theta/\rho gR$ when: the surface tension of the liquid is represented by $\gamma$, the contact angle is represented by $\theta$, the gravity acceleration is represented by g, and the density of the liquid is represented by $\rho$. This equation is led from both equations of equation (2): $p = 2\gamma\cos\theta/Rc$ (Laplace equation) and equation (5): $Leq = p/\rho g$ as described on p. 36 and p. 37 respectively of "ABSORBENCY" (ELSEVIER) edited by P. K. Chatterjee, but Leq is transcribed into h, and Rc is transcribed into R.

As to the apparatus of Fig. 1, if the head difference between the tank and the measurement cell is lifted from 0 to h (cm), then water as retained in openings having radii larger than the capillary radius (opening) R ($\mu$m) in a liquid existing either between particles of a swollen gel or in openings of the absorbent structure is gradually released to get out of them. Accordingly, if the height of a gel of which the opening spaces have been filled entirely with the liquid as a result of saturation-swelling is gradually raised from 0 cm to measure the amount of the residual liquid in the openings of the gel layer at each predetermined height, then the distribution of the space radii (capillary radii) in the swollen gel can be determined.

Hereinafter, in the present invention, the value of the capillary radius R, which is determined from the equation: $h = 2\gamma\cos\theta/\rho gR$ at each height h, of the sample is defined as the space radius of the sample. If the head difference between the tank and the measurement cell is lifted from 0 to 60 (cm) stepwise such as 1 cm, 2 cm, 5 cm, 10 cm, 20 cm, 30 cm, 60 cm, then a liquid as retained in openings having a value of R corresponding to each height is gradually discharged. If the amount of this liquid as discharged is measured, then the distribution of the space radii (capillary radii) of the sample can be calculated, and its values are plotted on logarithmic probability paper to define the value of d50 as the average space radius.

Incidentally, in the present invention, as the constants in the equation: $h = 2\gamma\cos\theta/\rho gR$, there are used the following values: $\gamma$: surface tension of physiological saline (0.9 mass % aqueous NaCl solution) (0.0728 kg/s$^2$), $\theta$: contact angle (0°), $\rho$: density of physiological saline (0.9 mass % aqueous NaCl solution) (1,000 kg/m$^3$), g: gravity acceleration 9.8 m/s$^2$. Thereby it is determined that a liquid as retained in the positions of 1 cm, 2 cm, 5 cm, 10 cm, 20 cm, 30 cm, 60 cm is retained at the space radii (capillary radii) of 1,485 $\mu$m, 743 $\mu$m, 297 $\mu$m, 149 $\mu$m, 74.3 $\mu$m, 49.5 $\mu$m, 24.8 $\mu$m respectively as the maximum space radii.

1-1. Average space ratio and average space radius without load:

1) A conduit 3 is connected to a lower portion of a glass filter 2 of 60 mm in diameter having a liquid-absorbing surface of a porous glass plate 1 (glass filter particle No. #3: Buchner type filter TOP 17G-3 (code no. 1175-03) produced by Sogo Rikagaku Glass Seisakusho Co., Ltd.), and this conduit 3 is connected to an opening as provided to a lower portion of a liquid storage container 4 of 10 cm in diameter. The porous glass plate of the aforementioned glass filter has an average pore diameter of 20 to 30 $\mu$m, and can retain water in the porous glass plate by its capillary force against the negative pressure of the water column even in a state where a difference of 60 cm between heights of liquid surfaces is made, so that a state of no introduction of air can be kept. A supporting ring 5 is fitted to the glass filter 2 in order to let up and down its height, and the system is filled with a physiological saline (0.9 mass % aqueous NaCl solution) 6, and the liquid storage container is put on a balance 7. After it is confirmed that there is no air in the conduit and under the porous glass plate of the glass filter, the difference in height between a liquid surface level of the top of the physiological saline (0.9 mass % aqueous NaCl solution) 6 in the liquid storage container 4 and a level of the upside of the porous glass plate 1 is adjusted to 60 cm, and the glass filter is fixed to a stand 8, and the value of the balance is set at 0.

2) A specimen to be measured 9 (water-absorbent resin or absorbent structure) is put on the porous glass plate 1 in the following way.

In the case where the specimen to be measured 9 is a water-absorbent resin, about 0.9 g (W) of it is quickly dispersed uniformly onto the porous glass plate 1 of the glass filter.

In the case where the specimen to be measured 9 is an absorbent structure, a sample of it as stamped into the shape of a circle of 57 mm in diameter is prepared, and its mass (W) in a dry state is measured, and then the sample is put on the porous glass plate 1 to carry out the measurement.

3) The difference in height between a liquid surface level of the top of the physiological saline (0.9 mass % aqueous NaCl solution) 6 in the liquid storage container 4 and a level of the upside of the porous glass plate 1 is changed to -3 cm (the porous glass plate 1 is located in a lower position) to swell the specimen for 20 minutes, when the specimen is put in a state entirely immersed in the physiological saline (0.9 mass % aqueous NaCl solution) so as to be free of air bubbles.

4) The difference in height between a liquid surface level of the top of the physiological saline (0.9 mass % aqueous NaCl solution) 6 in the liquid storage container 4 and a level of the upside of the porous glass plate 1 is changed to 0 cm, and then the system is left alone for 40 minutes to thereby saturation-swell the specimen, when the value of the balance is recorded (A0). Incidentally, in the case of the specimen which does not saturation-swell in 40 minutes, the time is occasionally extended.

5) The difference in height between a liquid surface level of the top of the physiological saline (0.9 mass % aqueous NaCl solution) 6 in the liquid storage container 4 and a level of the upside of the porous glass plate 1 is changed to 1 cm, and then, 7 minutes later, the value of the balance is recorded (A1). Dependently on the space diameter of the specimen, occasionally, this equilibrium time of till water as retained in openings is discharged had better be extended.

6) Similarly, the difference in height between a liquid surface level of the top of the physiological saline (0.9 mass % aqueous NaCl solution) 6 in the liquid storage container 4 and a level of the upside of the porous glass plate 1 is gradually raised to 2 cm, 5 cm, 10 cm, 20 cm, 30 cm, 60 cm, and then, 7 minutes later each, their respective values of the balance are recorded (A2, A5, A10, A20, A30, A60).

7) In order to entirely remove water as retained in openings at the difference of 60 cm in height between a liquid surface level of the top of the physiological saline (0.9 mass % aqueous NaCl solution) 6 in the liquid storage container 4 and a level of the upside of the porous glass plate 1, the specimen is got out and then subjected to centrifugal separation (250 G, 6 minutes), and then its mass B is measured.

8) (A0 - B) is the amount of water which fills the entire openings in the specimen (amount of water in the entire openings), and the values as left by subtracting the value of B from (A1, A2, A5, A10, A20, A30, A60) are the amounts of water as retained in openings by the capillary suction force of openings between particles against the negative pressure of the water column at the heights of 1 cm, 2 cm, 5 cm, 10 cm, 20 cm, 30 cm, 60 cm respectively (amounts of water as retained in openings). As previously mentioned, it is determined that a liquid as retained in the positions of 1 cm, 2 cm, 5 cm, 10 cm, 20 cm, 30 cm, 60 cm is retained at the space radii (capillary radii) of 1,485 μm, 743 μm, 297 μm, 149 μm, 74.3 μm, 49.5 μm, 24.8 μm respectively at the maximum. Therefore, the percentage (%) of the amount of water as retained in openings at each height relative to the amount of water in the entire openings (A0 - B) is calculated, and this value and the above value of the capillary radius are plotted on logarithmic probability paper (for one example, the value of (A2 - B)/(A0 - B) × 100 is plotted on 743 μm of the graph). A value (d50) of an space radius corresponding to 50 % of the amount as retained in openings on this graph is determined and defined as the average space radius (μm).

9) The average space ratio is determined from the following equation.

$$\text{Average space ratio} = [(A0 - B)/\{A0 + W/(\text{true density of sample})\}] \times 100$$

10) In order to further confirm the measured values, spherical glass beads of 350 to 500 μm and 1,000 to 1,180 μm were used as standard samples to determine the average space radii by the present method. As a result, they were determined as 86 μm and 217 μm respectively.

1-2. Average space ratio and average space radius under load of 2.07 kPa (0.3 psi):

Of the procedures 1) to 9) of the above measurement method 1-1, only the procedure 4) is modified into the following 4'). However, in the case where there is a possibility that the hot-melt adhesive of the absorbent structure may adhere due to such as load, a releasable base material of which the water absorbency is ignorable may be interposed.

4') The specimen is put on the porous glass plate of the glass filter, and further thereon a load (10) of 2.07 kPa (0.3 psi) having a diameter of 59 cm is put, and the difference in height between a liquid surface level of the top of the physiological saline (0.9 mass % aqueous NaCl solution) 6 in the liquid storage container 4 and a level of the upside of the porous glass plate 1 is changed to 0 cm, and then, 60 minutes later, the value of the balance is recorded (A0).

2. Capillary absorption capacity at height of 20 cm:

The capillary absorption capacity in the present invention is determined by measuring the amount of a liquid as absorbed by a specimen, namely, an absorbent structure or a water-absorbent resin, without load (under a load of 0.06 psi dependently on the measurement instrument) within a predetermined time against the negative pressure of the water column of 20 cm. The apparatus for measuring the capillary absorption capacity is shown in Fig. 1, and the measurement method with this apparatus is described below.

1) A conduit 3 is connected to a lower portion of a glass filter 2 of 60 mm in diameter having a liquid-absorbing surface of a porous glass plate 1 (glass filter particle No. #3: Buchner type filter TOP 17G-3 (code no. 1175-03) produced by Sogo Rikagaku Glass Seisakusho Co., Ltd.), and this conduit 3 is connected to an opening as provided to a lower portion of a liquid storage container 4 of 10 cm in diameter. The porous glass plate of the aforementioned glass filter has an average pore diameter of 20 to 30 μm, and can retain water in the porous glass plate by its capillary force against the negative pressure of the water column even in a state where a difference of 60 cm between heights of liquid surfaces is made, so that a state of no introduction of air can be kept. A supporting ring 5 is fitted to the glass filter 2 in order to let up and down its height, and the system is filled with a physiological saline (0.9 mass % aqueous NaCl solution) (0.9 % NaCl solution) 6, and the liquid storage container is put on a balance 7. After it is confirmed that there is no air in the conduit and under the porous glass plate of the glass filter, the difference in height between a liquid surface level of the top of the physiological saline (0.9 mass % aqueous NaCl solution) 6 in the liquid storage container 4 and a level of the upside of the porous glass plate 1 is adjusted to 20 cm, and the glass filter is fixed to a stand 8.
2) A specimen to be measured 9 (water-absorbent resin or absorbent structure) is put on the porous glass plate 1 under the following conditions, and further thereon a load 10 (0.06 psi) having a diameter of 59 mm is put, and then, 30 minutes later, there is measured a value (W20) of the physiological saline (0.9 mass % aqueous NaCl solution) as absorbed by the specimen to be measured 9.

In the case where the specimen to be measured 9 is an absorbent structure, a sample of it as stamped into the shape of a circle of 57 mm in diameter is prepared, and its mass (Wi) in a dry state is measured, and then the sample is put on the porous glass plate 1 to carry out the measurement.

In the case where the specimen to be measured 9 is a water-absorbent resin, 0.44 g of it is quickly dispersed uniformly onto the glass filter in the funnel.

The capillary absorption capacity in the present invention is determined from the following equations.

a) Capillary absorption capacity (g/g) of absorbent structure at height of 20 cm

= absorption amount (W20) (g)/mass (Wi) (g) of specimen to be measured before liquid absorption

b) Capillary absorption capacity D1 (g/g) of water-absorbent resin at height of 20 cm

= absorption amount (W20) (g)/0.44 (g)

3. Absorption capacity:

About 0.20 g (Wp1) of water-absorbent resin was placed uniformly into a nonwoven-fabric-made bag (60 × 60 mm) and then immersed into a 0.9 mass % aqueous sodium chloride solution (physiological saline). After 60 minutes, the bag was pulled up and then drained of water at 250 G with a centrifugal machine for 3 minutes, and then the resultant mass Wa (g) was measured. In addition, the same procedure as above was carried out without using the water-absorbent resin, and the resultant mass Wb (g) was measured. Then, from these masses Wa and Wb, the absorption capacity (g/g) of the water-absorbent resin was calculated in accordance with the following equation.

Absorption capacity (g/g)

= [mass Wa (g) - mass Wb (g)]/mass Wp1 (g) of water-absorbent resin

4. Absorption capacity under load:

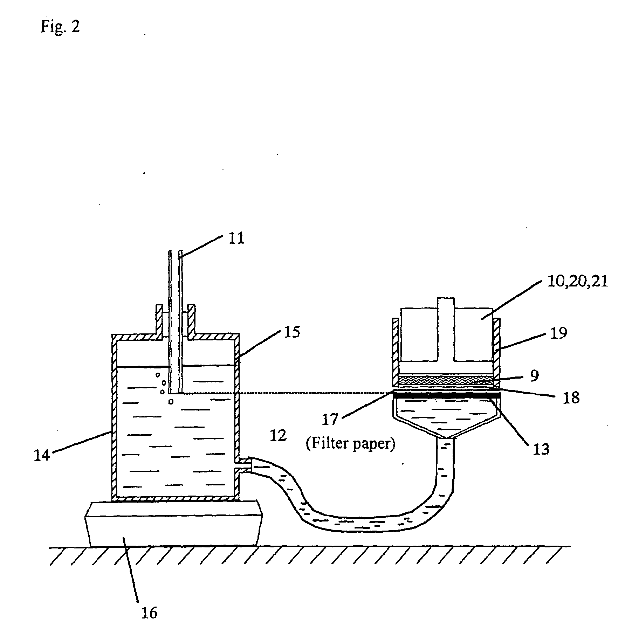

The absorption capacity under a load is measured with an apparatus of Fig. 2. There were prepared a load 20 and a load 21 as adjusted so as to give a pressure of 2.07 kPa (0.3 psi) and a pressure of 4.83 kPa (0.7 psi) respectively. Onto a metal gauze 18 of 400 meshes (mesh opening size of 38 μm) of a plastic cylinder 19 of 60 mm in diameter with the metal gauze stuck on its bottom, there is dispersed about 0.44 g (Wp2) of water-absorbent resin, and further thereon the above load 20 (in the case of 2.07 kPa (0.3 psi)) or load 21 (in the case of 0.7 psi) are put to prepare a liquid absorption instrument, which is then put on a filter paper 17 on a glass filter 13 of Fig. 2. After 30 minutes, there is measured a value (Wc) of the physiological saline (0.9 mass % aqueous NaCl solution) as absorbed by the water-absorbent resin. The absorption capacity under each load of 2.07 kPa (0.3 psi) and 4.83 kPa (0.7 psi) is determined using the following equation.

Absorption capacity (g/g) under load = Wc/Wp2

5. Average particle diameter:

The water-absorbent resin was classified with sieves of 850 μm, 600 μm, 500 μm, 300 μm, 150 μm, 75 μm, 45 μm in mesh opening size (if necessary, further a JIS standard sieve is added) to determine the ratio by mass of residual resin on each sieve, and then the residual percentage R was plotted on logarithmic probability paper, and the particle diameter corresponding to R = 50 % was defined as the average particle diameter.
The average particle diameter as obtained by the above measurement method is a mass-average particle diameter based on the mesh opening diameters of the sieves.

6. Method for evaluation of performances of absorbent structure and absorbent article:

6-1. Fixation ratio of swollen gel of absorbent structure:

A blended absorbent structure, including a water-absorbent resin and pulp, was got out of a commercially available diaper for children, and then an absorbent structure (12 cm × 38 cm) (as obtained in an Example mentioned below) was instead inserted to obtain an absorbent article. Then, 150 g of physiological saline (0.9 mass % aqueous NaCl solution) was injected into a central portion of this absorbent article, and then it was fitted to an infant doll, and then both its legs were moved by 100 steps in imitation of walking motion, and then the absorbent article was put off. The top sheet of the diaper was removed, and then the diaper was left hung down for 1 minute in a state where the absorbent structure was on the downside. Then, the mass (Wb) of the water-absorbent resin having fallen off from the internal absorbent structure was measured to determine the fixation ratio of the swollen gel in accordance with the following equation.

Fixation ratio (%) of swollen gel = [{150 (g) - mass Wb (g) having fallen off}/150 (g)] × 100

6-2. Plane absorbency test of model absorbent article:

Method for preparation of model absorbent article (for plane):

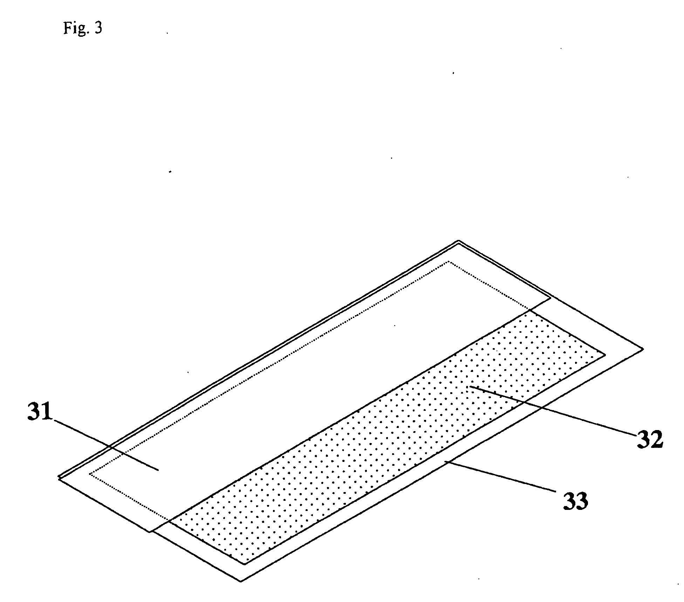

Onto the upside of an absorbent structure (12 cm × 38 cm) (as obtained in each Example mentioned below), there was dispersed a blue-colored water-absorbent resin as a hydrophilic release material at a basis weight of 10 g/m$^2$, and thereon a rectangular polyester nonwoven fabric of 12 × 40 cm (basis weight: 20 g/m$^2$, thickness: 0.28 mm) was put as a liquid-permeable surface material to obtain the model absorbent article (for plane) (Fig. 3).

Method for plane absorbency test:

The above model absorbent article was fixed onto a desk with a tape in the shape of a plane, and then there were applied thereto an acrylic plate of 12 × 40 cm (having, in its central portion, a cylinder of 70 mm in diameter for liquid injection) and a load of 1.3 kg.

① Liquid permeation time:

An amount of 75 ml of a red-colored physiological saline (0.9 mass % aqueous NaCl solution), as adjusted to 37 °C, is poured into the cylinder to measure a time for the liquid to completely be absorbed from the surface sheet into the absorbent article (first-time liquid permeation time).

② Diffusing ability:

After 60 minutes subsequently to the above measurement, there is measured an area (S cm$^2$) of liquid diffusion from the upside of the liquid-permeable surface material into the absorbent structure.
The area of diffusion in the absorbent article according to the present invention can be determined from the following equation.

Area (%) of diffusion

= area (S cm$^2$) of liquid diffusion/area (12 × 38 cm) of absorbent structure

③ Wet-back quantity:

The above procedure is repeated four times at intervals of 60 minutes (in these repeated procedures, the second-time to fourth-time liquid permeation times are measured), and then the absorbent article is left alone for 60 minutes after the fourth-time injection. After 60 minutes, the acrylic plate is removed, and then 15 pieces of kitchen paper (Nepia of Oji Paper-Manufacturing Co., Ltd., 46 × 22 cm) are piled so as to cover the upside of the absorbent article, and then a load of 12 kg (including an acrylic plate of 14 × 40 cm) is applied for 1 minute to measure the quantity of the liquid going back to the kitchen paper, and this measured quantity is regarded as the wet-back quantity.

6-3. Inclination absorbency test of model absorbent article:

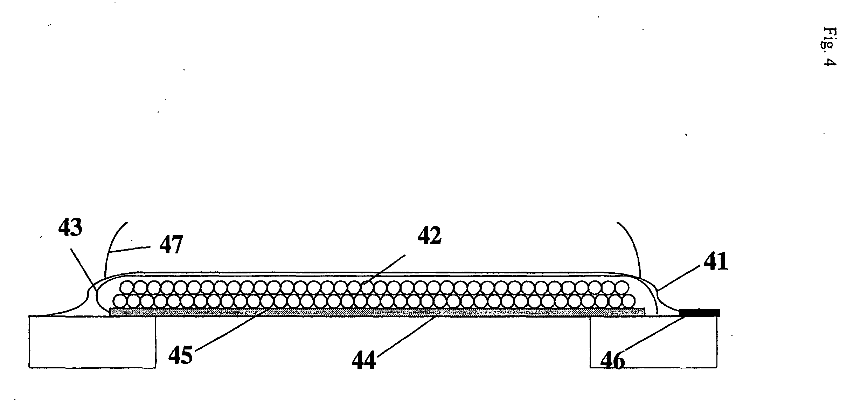

Method for preparation of model absorbent article (for inclination): with reference to Fig. 4

Merries Morenai Fit (produced by Kao Corporation; size L), which was a commercially available disposable diaper, was used. An absorbent structure portion was removed from an edge of one side of the diaper, and then a liquid-permeable surface material (top sheet), a liquid-impermeable material (back sheet), and a diffusing paper were cut out as test pieces.
Onto the liquid-impermeable material back sheet (size: 14 cm in width × 43 cm in length), there was dispersed about 1 g of a solvent type pressure sensitive adhesive (acrylic resin spray paste, produced by Sekisui Chemical Co., Ltd.). Next, thereon a water-absorbent resin was dispersed so as to be 12 cm in width, 38 cm in length, and 360 g/m$^2$ in basis weight (16.4 g), and further onto it, about 3 g of ion-exchanged water was mistwise sprayed to temporarily fix the resin. Next, the liquid-permeable surface material (size: 14 cm in width × 43 cm in length) with the diffusing paper was put on the upper layer of the water-absorbent resin, and then the top sheet and the back sheet were stuck to each other at the sides of the absorbent structure with an adhesive (paper double-coated tape, Nice Tack, produced by Nichiban Co., Ltd.), and then they were left alone under a temperature of 25 °C and a humidity of 60 % RH for 16 hours, thus preparing the model absorbent article (for inclination).

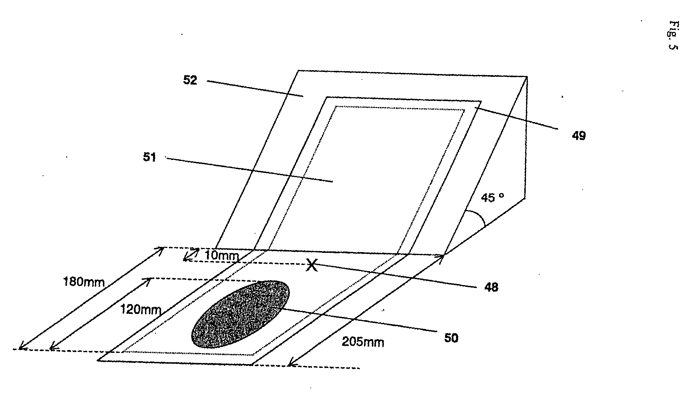

Method for inclination absorbency test: with reference to Fig. 5

Each of the model absorbent articles, as obtained in the below-mentioned Examples and Comparative Examples, was evaluated by absorption performances (absorption rate, diffusion area, maximum absorption quantity) in the following ways.
As is shown in Fig. 5, there was prepared an L-character-shaped instrument having an inclined-plane stand of 45° in angle (width: 20 cm, length: 30 cm), and thereon the model absorbent article was put and fixed with a tape. The L-character-shaped instrument inclined at 45° from a position of 205 mm away from an end of a plane portion (a position of 180 mm away from an end of the absorbent structure), and a weight of 4.43 kg (pressing face: 73 cm$^2$) was put on the range of 10 mm to 120 mm away from an end of the model absorbent article on the plane portion, and the absorption performances were evaluated. This state assumes a state of a diaper worn by a child lying prone.

In this assumed state of lying prone, 75 ml of a red-colored physiological saline, as adjusted to 37 °C, is injected from a position of 195 mm away from an end of the plane portion (a position of 170 mm away from an end of the belly side of the absorbent structure) at a rate of 15 g/second to measure a time for the physiological saline to completely be absorbed from the top sheet into the model absorbent article (first-time liquid permeation time, unit: second). The physiological saline is additionally injected at intervals of 1 hour to measure each liquid permeation time. The additional injection of the physiological saline is continued till the liquid leaks from an end of the model absorbent article on the plane portion. The state of the liquid absorption is observed by the eye measurement to measure a time for the liquid to come into a state of non-residence on the top sheet as a result of the absorption into the absorbent structure, and this measured time is defined as the liquid permeation time. In addition, there is measured a time for the top sheet to, as a result of further absorption, come into a state of indicating its own white color without indicating the red color of the physiological saline, and this measured time is defined as the whitening time. The addition of the liquid is continued till the liquid leaks from an end on the plane portion and, if the leakage occurs, the addition of the liquid is stopped, and then the system is left alone for 1 hour. During this leaving alone, the physiological saline residing at the end on the plane portion is removed by keeping a state where a pile of five pieces of Kim Towel Wiper (produced by Cresia Co., Ltd.; about 19 × 16 cm) are in contact with the end on the plane portion. After 1 hour, the mass of the absorbent structure is measured, and then the liquid-permeable surface is removed to measure an area (S cm$^2$) of diffusion of the physiological saline into the absorbent structure.

The physical properties in the model absorbent article according to the present invention can be determined from the following equations.

$$\text{Area (\%) of diffusion}$$

$$= \text{area (S cm}^2\text{) of liquid diffusion/area (12} \times \text{38 cm) of absorbent structure}$$

$$\text{Maximum absorption quantity (g)}$$

$$= \text{final mass of diaper - mass of diaper before liquid absorption}$$

$$\text{Liquid permeation rate (ml/sec)}$$

$$= \text{total liquid absorption quantity before occurrence of leakage}$$

$$\text{/total absorption rate before occurrence of leakage}$$

$$\text{Whitening property (at third-time liquid addition) (ml/min)} = 75 \text{ (ml)/whitening time (min)}$$

7. Method for measurement of number-average fiber diameter and number-average diameter as to intervals between fibers of hot-melt adhesive fiber web:

The absorbent structure, as fixed with the hot-melt adhesive, was observed and photographed at 150 magnifications with a digital optical microscope (product name: Portable Digital Microscope YH-6300C, produced by Keyence Co., Ltd.).

From the taken photograph of the absorbent structure, 20 pieces of hot-melt adhesive fibers were selected to measure their respective maximum fiber diameters, and their average value was defined as the number-average fiber diameter of the hot-melt adhesive. Similarly, 20 fiber meshes were selected to measure their respective maximum intervals, and their average value was defined as the number-average diameter as to intervals between fibers.

## DETAILED DESCRIPTION OF THE PREFERRED EMBODIMENTS

Hereinafter, the present invention is more specifically illustrated by the following examples of some preferred embodiments in comparison with comparative examples not according to the invention. However, the invention is not limited thereto in any way.

[Referential Example 1] production process for water-absorbent resin (1):

A reaction solution was prepared by dissolving 8.1 parts of polyethylene glycol diacrylate (n = 8) into 5,500 parts of a 38 mass % aqueous solution of sodium acrylate (neutralization ratio: 71 mol %). Next, this reaction solution was deaerated under a nitrogen gas atmosphere for 30. Next, the reaction solution was supplied to a jacketed stainless-steel-made twin-arm kneader having two sigma type blades along with a lid possible to open and close. While the reaction solution was kept at 30 °C, air in the system was displaced with nitrogen gas. Subsequently, while the reaction solution was stirred, 2.4 parts of ammonium persulfate and 0.12 part of L-ascorbic acid were added thereto. As a result, polymerization started after about 1 minute. Then, the polymerization was carried out in the range of 20 to 95 °C, and the resultant hydrogel polymer was got out 60 minutes later than the start of the polymerization.

The resultant hydrogel polymer was a finely divided one having a diameter of about 5 mm. This finely divided hydrogel polymer was spread onto a 50-mesh metal gauze and then dried with hot air of 150 °C for 90 minutes. Next, the dried product was pulverized with a vibratory mill, and then there was obtained an irregular pulverized polymer (1) of 400 μm in average particle diameter which passed through a sieve of 850 μm in mesh opening size but remained on a sieve of 106 μm in mesh opening size.

An amount of 100 mass parts of the resultant polymer (1) was mixed with a surface-crosslinking agent composition liquid including 0.4 mass part of 1,4-butanediol, 0.5 mass part of propylene glycol, and 3 mass parts of water. The resultant mixture was heat-treated at 210 °C for 40 minutes to obtain the water-absorbent resin (1). The average particle diameter of the water-absorbent resin (1) was 410 μm. The capillary absorption capacity of the water-absorbent resin (1) at 20 cm was 21 (g/g). In addition, the average space ratios and average space radii of the water-absorbent resin (1) during saturation-swelling without load and under 2.07 kPa (0.3 psi) were determined to show their results in Table 1.

[Referential Example 2] production process for water-absorbent resin (2):

A reaction solution was prepared by dissolving 5 parts of polyethylene glycol diacrylate (n = 8) into 5,500 parts of a 33 mass % aqueous solution of sodium acrylate (neutralization ratio: 75 mol %). Next, this reaction solution was deaerated under a nitrogen gas atmosphere for 30. Next, the reaction solution was supplied to a jacketed stainless-steel-made twin-arm kneader having two sigma type blades along with a lid possible to open and close. While the reaction solution was kept at 30 °C, air in the system was displaced with nitrogen gas. Subsequently, while the reaction solution was stirred, 2.4 parts of ammonium persulfate and 0.12 part of L-ascorbic acid were added thereto. As a result, polymerization started after about 1 minute. Then, the polymerization was carried out in the range of 30 to 90 °C, and the resultant hydrogel polymer was got out 60 minutes later than the start of the polymerization.

The resultant hydrogel polymer was a finely divided one having a diameter of about 5 mm. This finely divided hydrogel polymer was spread onto a 50-mesh metal gauze and then dried with hot air of 150 °C for 90 minutes. Next, the dried product was pulverized with a vibratory mill and then classified with a 20-mesh metal gauze to obtain an irregular pulverized polymer (2) which had an average particle diameter of 360 μm and of which 3 mass % of the particles had particle diameters of smaller than 106 μm.

An amount of 100 mass parts of the resultant polymer (2) was mixed with a surface-crosslinking agent composition liquid including 0.08 mass part of ethylene glycol diglycidyl ether, 0.5 mass part of glycerol, 3 mass parts of water, and 1 mass part of isopropyl alcohol. The resultant mixture was heat-treated at 195 °C for 40 minutes to obtain a water-absorbent resin (2'). The resultant water-absorbent resin (2') was further passed through a metal gauze of 250 μm in mesh opening size to obtain the water-absorbent resin (2) under the sieve. The average particle diameter of the water-absorbent resin (2) was 120 μm. The capillary absorption capacity of the water-absorbent resin (2) at 20 cm was 27 (g/g). In addition, the average space ratios and average space radii of the water-absorbent resin (2) during saturation-swelling without load and under 2.07 kPa (0.3 psi) were determined to show their results in Table 1.

[Referential Example 3] production process for (comparative) water-absorbent resin (3):

An amount of 100 mass parts of the water-absorbent resin (1) was mixed with 0.8 mass part of polyethylenimine as a cationic polymer to obtain the water-absorbent resin (3). The average particle diameter of the water-absorbent resin (3) was 500 μm. In addition, the capillary absorption capacity of the water-absorbent resin at 20 cm was 12 (g/g). The average space ratios and average space radii of the water-absorbent resin (3) during saturation-swelling without load and under 2.07 kPa (0.3 psi) were determined to show their results in Table 1.

[Referential Example 4] production process for water-absorbent resin (4):

A reaction solution was prepared by dissolving 5.8 parts of polyethylene glycol diacrylate (n = 8) into 5,500 parts of a 38 mass % aqueous solution of sodium acrylate (neutralization ratio: 71 mol %). Next, this reaction solution was deaerated under a nitrogen gas atmosphere for 30. Next, the reaction solution was supplied to a jacketed stainless-steel-made twin-arm kneader having two sigma type blades along with a lid possible to open and close. While the reaction solution was kept at 30 °C, air in the system was displaced with nitrogen gas. Subsequently, while the reaction solution was stirred, 3.0 parts of sodium persulfate and 0.03 part of L-ascorbic acid were added thereto. As a result, polymerization started after about 1 minute. Then, the polymerization was carried out in the range of 20 to 95 °C, and the resultant hydrogel polymer was got out 60 minutes later than the start of the polymerization. The resultant hydrogel polymer was a finely divided one having a diameter of about 5 mm. This finely divided hydrogel polymer was spread onto a 50-mesh metal gauze and then dried with hot air of 150 °C for 90 minutes. Next, the dried product was pulverized with a vibratory mill, and then there was obtained an irregular pulverized polymer (4) of 200 μm in average particle diameter which passed through a sieve of 500 μm in mesh opening size but remained on a sieve of 45 μm in mesh opening size.

An amount of 100 mass parts of the resultant polymer (4) was mixed with a surface-crosslinking agent composition liquid including 0.4 mass part of 1,4-butanediol, 0.5 mass part of propylene glycol, and 3 mass parts of water. The resultant mixture was heat-treated at 210 °C for 40 minutes, and then thereto 0.5 mass part of a fine powder of hydrophilic silicon dioxide (Aerosil 200, produced by Nippon Aerosil Co., Ltd.) was added to coat it to the surface portions to obtain the water-absorbent resin (4). The average particle diameter of the water-absorbent resin (4) was 218 μm. In addition, the capillary absorption capacity of the water-absorbent resin (4) at 20 cm was 24.6 (g/g). In addition, the average space ratios and average space radii of the water-absorbent resin (4) during saturation-swelling without load and under 2.07 kPa (0.3 psi) were determined to show their results in Table 2.

[Referential Example 5] production process for water-absorbent resin (5):

The polymer (4), as obtained in Referential Example 4, was further classified with a 50-mesh metal gauze to obtain an irregular pulverized polymer (5) which had an average particle diameter of 120 μm. The resultant polymer (5) was mixed with the surface-crosslinking agent composition and then heat-treated in the same way as of Referential Example 4, and then thereto 0.5 mass part of bentonite (produced by Wako Pure Chemical Industries, Ltd. (catalogue code 029-0055)) was added to mix them to obtain the water-absorbent resin (5).

The average particle diameter of the water-absorbent resin (5) was 125 μm. The capillary absorption capacity of the water-absorbent resin (5) at 20 cm was 22.8 (g/g). In addition, the average space ratios and average space radii of the water-absorbent resin (5) during saturation-swelling without load and under 2.07 kPa (0.3 psi) were determined to show their results in Table 2.

[Referential Example 6] production process for (comparative) water-absorbent resin (6):

An amount of 0.5 mass part of Aerosil 200 (produced by Nippon Aerosil Co., Ltd.) was added to 100 of the water-absorbent resin (1) to mix them to obtain the water-absorbent resin (6). The average particle diameter of the water-absorbent resin (6) was 425 μm. In addition, the capillary absorption capacity of the water-absorbent resin at 20 cm was 11 (g/g). The average space ratios and average space radii of the water-absorbent resin (6) during saturation-swelling without load and under 2.07 kPa (0.3 psi) were determined to show their results in Table 2.

[Referential Example 7] liquid-acquiring member:

As the liquid-acquiring member, crosslinked cellulose (being in a state its upside and downside were covered with nonwoven fabric) was got out of a commercially available disposable diaper (Pampers Sara-Sara Care, produced by P&G, size: L, mass of diaper: 57 g) and then used as the liquid-acquiring member in the size of 8 cm × 24 cm.

[Example 1]:

An SBS type hot-melt adhesive (Hi-Bon 9612, produced by Hitachi Chemical Polymer Co., Ltd., melt viscosity at 180 °C: 1,300 mPa·s, softening point: 88 °C) was melted with a hot-melt-coating apparatus (body: Dynapro 2000GP-model hot-melt applicator, coating nozzle: Dynafiber UFD of a type having 14 openings, both were products of ITW Dynatec) of which the temperature had been raised to 180°C.

Next, a paper base material of 15 × 50 cm (basis weight: 22 g/m$^2$, thickness: 0.07 mm) was set as a base material on a belt conveyor, and then the above SBS type hot-melt adhesive was coated onto this base material so as to be 12.5 cm in width, 40 cm in length, and 10 g/m$^2$ in basis weight while the belt conveyor was run. Next, onto the adhesive, there was dispersed the water-absorbent resin (1) so as to be 12 cm in width, 38 cm in length, and 360 g/m$^2$ in basis weight, and then thereonto about 3 g of ion-exchanged water was sprayed to temporarily fix the resin.

While this paper base material and the resultant layer including the water-absorbent resin (1) were run on the belt conveyor again, the above SBS type hot-melt adhesive was dispersed onto the water-absorbent resin so as to be 15 cm in width, 40 cm in length, and 10 g/m$^2$ in basis weight again, thus obtaining an absorbent structure (1). The hot-melt adhesive, coating the upper layer of the water-absorbent resin, was in the shape of a finely fibrous web net. The hot-melt adhesive fibers had a number-average fiber diameter of 21 μm and a number-average diameter of 71 μm as to intervals between fibers. This finely-fibrous-web-shaped hot-melt adhesive had an elongation of not less than 500 %. The average space ratios and average space radii of the absorbent structure (1) during saturation-swelling without load and under 2.07 kPa (0.3 psi) were determined to show their results in Table 1.

From the resultant absorbent structure (1), an absorbent article (1) was prepared in the aforementioned way.

Furthermore, onto the absorbent structure (1), there was dispersed the blue-colored water-absorbent resin (1) as a hydrophilic release material at a basis weight of 10 g/m$^2$, and thereon a rectangular polyester nonwoven fabric of 12 × 40 cm (basis weight: 20 g/m$^2$, thickness: 0.28 mm) was put as a liquid-permeable surface material to obtain a model absorbent article for plane (1). The fixation ratio of the swollen gel was measured using the absorbent article (1), and the model absorbent article for plane (1) was evaluated by the liquid permeation time, the area of diffusion, and the wet-back quantity in the above evaluation ways, and their results are shown in Table 1.

[Example 2]:

An SBS type hot-melt adhesive (Hi-Bon 9612, produced by Hitachi Chemical Polymer Co., Ltd., melt viscosity at 180 °C: 1,300 mPa·s, softening point: 88 °C) was melted with a hot-melt-coating apparatus (body: Dynapro 2000GP-model hot-melt applicator, coating nozzle: Dynafiber UFD of a type having 14 openings, both were products of ITW Dynatec) of which the temperature had been raised to 180°C.

Next, a paper base material of 15 × 50 cm (basis weight: 22 g/m$^2$, thickness: 0.07 mm) was set as a second base material on a belt conveyor, and then the above SBS type hot-melt adhesive was coated onto this second base material so as to be 12.5 cm in width, 40 cm in length, and 10 g/m$^2$ in basis weight while the belt conveyor was run. Further onto the second base material, there was press-attached a thick polyester nonwoven fabric (produced by Kanai Juyo Kogyo Co., Ltd., basis weight: 30 g/m$^2$, fiber diameter: 6 deniers, thickness: 0.9 mm, gas permeability: 675.0 ml/cm$^2$/s, incidentally the gas permeability was measured in accordance with JIS L 1084) as cut into a width of 12 cm and a length of 38 cm as a first base material.

Next, onto the polyester nonwoven fabric as this first base material, there was dispersed the water-absorbent resin (1) so as to be 12 cm in width, 38 cm in length, and 360 g/m$^2$ in basis weight, and then thereonto about 3 g of ion-exchanged water was sprayed to temporarily fix the resin. While these first and second base materials and the resultant layer including the water-absorbent resin (1) were run on the belt conveyor again, the above SBS type hot-melt adhesive was dispersed onto the water-absorbent resin so as to be 15 cm in width, 40 cm in length, and 10 g/m$^2$ in basis weight again, thus obtaining an absorbent structure (2). The hot-melt adhesive, coating the upper layer of the water-absorbent resin, was in the shape of a finely fibrous web net. The hot-melt adhesive fibers had a number-average fiber diameter of 24 μm and a number-average diameter of 80 μm as to intervals between fibers. This finely-fibrous-web-shaped hot-melt adhesive had an elongation of not less than 500 %. The average space ratios and average space radii of the absorbent structure (2) during saturation-swelling without load and under 2.07 kPa (0.3 psi) were determined to show their results in Table 1.

Onto the resultant absorbent structure (2), there was dispersed the blue-colored water-absorbent resin (1) as a hydrophilic release material at a basis weight of 10 g/m$^2$, and thereon a rectangular polyester nonwoven fabric of 12 × 40 cm (basis weight: 20 g/m$^2$, thickness: 0.28 mm) was put as a liquid-permeable surface material to obtain a model absorbent article for plane (2). The fixation ratio of the swollen gel of the absorbent structure (2) was measured using the absorbent article (2), and the model absorbent article for plane (2) was evaluated by the liquid permeation time, the area of diffusion, and the wet-back quantity in the above evaluation ways, and their results are shown in Table 1.

[Example 3]:

An absorbent structure (3) was obtained in the same way as of Example 2 except that the water-absorbent resin (1) was replaced with the water-absorbent resin (2). The hot-melt adhesive, coating the upper layer of the water-absorbent resin, was in the shape of a finely fibrous web net. The hot-melt adhesive fibers had a number-average fiber diameter of 20 μm and a number-average diameter of 73 μm as to intervals between fibers. This finely-fibrous-web-shaped hot-melt adhesive had an elongation of not less than 500 %. The average space ratios and average space radii of the absorbent structure (3) during saturation-swelling without load and under 2.07 kPa (0.3 psi) were determined to show their results in Table 1.

From the resultant absorbent structure (3), an absorbent article (3) was prepared in the aforementioned way.

Onto the resultant absorbent structure (3), there was dispersed the blue-colored water-absorbent resin (2) as a hydrophilic release material at a basis weight of 10 g/m$^2$, and thereon a rectangular polyester nonwoven fabric of 12 × 40 cm (basis weight: 20 g/m$^2$, thickness: 0.28 mm) was put as a liquid-permeable surface material to obtain a model absorbent article for plane (3). In the above evaluation ways, the fixation ratio of the swollen gel of the absorbent structure (3) was evaluated using the absorbent article (3), and the model absorbent article for plane (3) was evaluated by the liquid permeation time, the area of diffusion, and the wet-back quantity. Their results are shown in Table 1.

[Example 4]:

An SBS type hot-melt adhesive (Hi-Bon 9612, produced by Hitachi Chemical Polymer Co., Ltd., melt viscosity at 180 °C: 1,300 mPa·s, softening point: 88 °C) was melted with a hot-melt-coating apparatus (body: Dynapro 2000GP-model hot-melt applicator, coating nozzle: Dynafiber UFD of a type having 14 openings, both were products of ITW Dynatec) of which the temperature had been raised to 180°C.

Next, a paper base material of 15 × 50 cm (basis weight: 22 g/m$^2$, thickness: 0.07 mm) was prepared, and about 1.5 g of ion-exchanged water was sprayed onto this base material. Next, thereonto, there was dispersed the water-absorbent resin (1) so as to be 12 cm in width, 38 cm in length, and 360 g/m$^2$ in basis weight, and then thereonto about 1.5 g of ion-exchanged water was sprayed to temporarily fix the resin. While this paper base material and the resultant layer including the water-absorbent resin (1) were run on the belt conveyor again, the above SBS type hot-melt adhesive was dispersed onto the water-absorbent resin so as to be 15 cm in width, 40 cm in length, and 10 g/m$^2$ in basis weight again, thus obtaining an absorbent structure (4). The hot-melt adhesive, coating the upper layer of the water-absorbent resin, was in the shape of a finely fibrous web net. The hot-melt adhesive fibers had a number-average fiber diameter of 21 μm and a number-average diameter of 73 μm as to intervals between fibers. This finely-fibrous-web-shaped hot-melt adhesive had an elongation of not less than 500 %. The average space ratios and average space radii of the absorbent structure (4) during saturation-swelling without load and under 2.07 kPa (0.3 psi) were determined to show their results in Table 1.

From the resultant absorbent structure (4), an absorbent article (4) was prepared in the aforementioned way.

Onto the resultant absorbent structure (4), there was dispersed the blue-colored water-absorbent resin (1) as a hydrophilic release material at a basis weight of 10 g/m$^2$, and thereon a rectangular polyester nonwoven fabric of 12 × 40 cm (basis weight: 20 g/m$^2$, thickness: 0.28 mm) was put as a liquid-permeable surface material to obtain a model absorbent article for plane (4). The fixation ratio of the swollen gel of the absorbent structure (4) was measured using the absorbent article (4), and the model absorbent article for plane (4) was evaluated by the liquid permeation time, the area of diffusion, and the wet-back quantity in the above evaluation ways, and their results are shown in Table 1.

[Comparative Example 1]:

A comparative absorbent structure (1) was obtained in the same way as of Example 1 except that the water-absorbent resin (1) was replaced with the water-absorbent resin (3). The hot-melt adhesive, coating the upper layer of the water-absorbent resin, was in the shape of a finely fibrous web net. The hot-melt adhesive fibers had a number-average fiber diameter of 22 μm and a number-average diameter of 75 μm as to intervals between fibers. This finely-fibrous-web-shaped hot-melt adhesive had an elongation of not less than 300 %. As is shown also in Table 1, the comparative absorbent structure (1) displayed an average space ratio of 53 % and an average space radius of 420 μm during saturation-swelling without load and displayed an average space ratio of 34 % and an average space radius of 138 μm during saturation-swelling under 2.07 kPa (0.3 psi), therefore its average space radii during saturation-swelling without load and under 2.07 kPa (0.3 psi) were out of the ranges according to the present invention.

From the resultant comparative absorbent structure (1), a comparative absorbent article (1) was prepared in the aforementioned way.

Onto the resultant comparative absorbent structure (1), there was dispersed the blue-colored water-absorbent resin (3) as a hydrophilic release material at a basis weight of 10 g/m$^2$, and thereon a rectangular polyester nonwoven fabric of 12 × 40 cm (basis weight: 20 g/m$^2$, thickness: 0.28 mm) was put as a liquid-permeable surface material to obtain a comparative model absorbent article (1). The fixation ratio of the swollen gel of the comparative absorbent structure (1) was measured using the comparative absorbent article (1), and the comparative model absorbent article (1) was evaluated by the liquid permeation time, the area of diffusion, and the wet-back quantity in the above evaluation ways, and their results are shown in Table 1. The comparative model absorbent article (1) displayed a large wet-back quantity and gave a uncomfortable feel of contact with the skin when used for such as diapers.

[Comparative Example 2]:

A comparative absorbent structure (2) was obtained in the same way as of Example 1 except that the water-absorbent resin (1) was replaced with the water-absorbent resin (2). The hot-melt adhesive, coating the upper layer

of the water-absorbent resin, was in the shape of a finely fibrous web net. The hot-melt adhesive fibers had a number-average fiber diameter of 21 μm and a number-average diameter of 68 μm as to intervals between fibers. This finely-fibrous-web-shaped hot-melt adhesive had an elongation of not less than 300 %. As is shown also in Table 1, the comparative absorbent structure (2) displayed an average space ratio of 43 % and an average space radius of 90 μm during saturation-swelling without load and displayed an average space ratio of 26 % and an average space radius of 45 μm during saturation-swelling under 2.07 kPa (0.3 psi), therefore its average space radii during saturation-swelling without load and under 2.07 kPa (0.3 psi) were out of the ranges according to the present invention.

From the resultant comparative absorbent structure (2), a comparative absorbent article (2) was prepared in the aforementioned way.

Onto the resultant comparative absorbent structure (2), there was dispersed the blue-colored water-absorbent resin (2) as a hydrophilic release material at a basis weight of 10 g/m$^2$, and thereon a rectangular polyester nonwoven fabric of 12 × 40 cm (basis weight: 20 g/m$^2$, thickness: 0.28 mm) was put as a liquid-permeable surface material to obtain a comparative model absorbent article (2). The fixation ratio of the swollen gel of the comparative absorbent structure (2) was measured using the comparative absorbent article (2), and the comparative model absorbent article (2) was evaluated by the liquid permeation time, the area of diffusion, and the wet-back quantity in the above evaluation ways, and their results are shown in Table 1. The comparative model absorbent article (2) displayed a long liquid permeation time and a slow absorption rate.

[Comparative Example 3]:

A comparative absorbent structure (3) was obtained in the same way as of Example 1 except that: the water-absorbent resin (1) was dispersed onto a paper base material of 15 × 50 cm (basis weight: 22 g/m$^2$, thickness: 0.07 mm), without using any hot-melt adhesive, so as to be 12 cm in width, 38 cm in length, and 360 g/m$^2$ in basis weight, and then about 3 g of ion-exchanged water was sprayed onto the resin. The comparative absorbent structure (3) was 0 % in the fixation ratio of the swollen gel and was therefore so easily deformed as to be inferior in the shape retainability.

[Examples 5, 6]:

From the water-absorbent resins (4) and (5) as obtained in Referential Examples 4 and 5, model absorbent articles (for inclination) (5) and (6) were prepared by the method for preparation of model absorbent article (for inclination) as specified in section 6-3 hereof. Then, their absorption rates, areas of diffusion, and maximum absorption quantities were evaluated, and their results are shown in Table 2.

[Example 7]:

An amount of 16.4 g of the water-absorbent resin, as obtained in Referential Example 4, is dispersed onto an area of 12 × 38 cm to form a water-absorbent resin layer of 360 g/m$^2$ in dispersion amount, and then thereon the liquid-acquiring member (12 × 24 cm, mass: 3.8 g) as obtained in Referential Example 7 is put to obtain a model absorbent article for inclination (7). The results are shown in Table 2. There can be understood to be obtained a diaper such that: the whitening time is also fast, and the amount of the residual liquid in the liquid-acquiring member is small, therefore the water-absorbent resin layer absorbs the liquid from the liquid-acquiring member to such a good extent that the feel of dryness is excellent.

[Comparative Examples 4, 5]:

From the water-absorbent resins as obtained in Production Examples 3 and 6, comparative model absorbent articles (4) and (5) were prepared in the same ways as of Examples 5 to 7. The comparative model absorbent articles for inclination (4) and (5) were evaluated by their absorption rates, areas of diffusion, and absorption quantities, and their results are shown in Table 2.

[Comparative Example 6]:

A comparative absorbent structure (6), including a combination of the water-absorbent resin (6) and the liquid-acquiring member (1), is obtained in the same way as of Example 5. Then, a comparative model absorbent article (6) was prepared in the same way as of Comparative Example 4 except to use the liquid-acquiring member. The results are shown in Table 2. The whitening time is slow, and the amount of the residual liquid in the liquid-acquiring member is also large, therefore it can be understood that there could not smoothly be done the absorption of the liquid from the liquid-acquiring member into the water-absorbent resin layer.

[Table 1] (part 1 of 2)

| | Example 1 | Example 2 | Example 3 | Comparative Example 1 | Comparative Example 2 | Comparative Example 3 |
|---|---|---|---|---|---|---|
| Base material | Paper base material | Nonwoven fabric+paper base material | Nonwoven fabric+paper base material | Paper base material | Paper base material | Paper base material |
| | | | | | | |
| Amount (g/m$^2$) of hot-melt adhesive as added | 10 | 10 | 10 | 10 | 10 | 0 |
| | | | | | | |
| Water-absorbent resin: | Water-absorbent resin (1) | Water-absorbent resin (1) | Water-absorbent resin (2) | Water-absorbent resin (3) | Water-absorbent resin (2) | Water-absorbent resin (1) |
| | | | | | | |
| absorption capacity (g/g) | 28 | 28 | 25 | 25 | 25 | 28 |
| absorption capacity (g/g) under load (0.3 psi) | 33 | 33 | 28 | 27 | 28 | 33 |
| absorption capacity (g/g) under load (0.7 psi) | 26 | 26 | 23 | 27 | 23 | 26 |
| average particle diameter (μm) | 410 | 410 | 120 | 500 | 120 | 410 |
| | | | | | | |
| without load: | | | | | | |
| average space ratio (%) of swollen gel | 39 | 39 | 43 | 53 | 43 | 39 |
| average space radius (μm) of swollen gel | 120 | 120 | 90 | 420 | 90 | 120 |
| under 0.3 psi: | | | | | | |
| average space ratio (%) of swollen gel | 23 | 23 | 25 | 34 | 25 | 23 |

[Table 1] (part 1 of 2)   (continued)

| | Example 1 | Example 2 | Example 3 | Comparative Example 1 | Comparative Example 2 | Comparative Example 3 |
|---|---|---|---|---|---|---|
| under 0.3 psi: | | | | | | |
| average space radius (µm) of swollen gel | 54 | 54 | 47 | 138 | 47 | 54 |
| | | | | | | |

[Table 1] (part 2 of 2)

| | Example 1 | Example 2 | Example 3 | Comparative Example 1 | Comparative Example 2 | Comparative Example 3 |
|---|---|---|---|---|---|---|
| Absorbent structure: | Absorbent structure (1) | Absorbent structure (2) | Absorbent structure (3) | Comparative absorbent structure (1) | Comparative absorbent structure (2) | Comparative absorbent structure (3) |
| without load: | | | | | | |
| average space ratio (%) of absorbent structure | 38 | 42 | 42 | 52 | 43 | - |
| average space radius (µm) of absorbent structure | 115 | 186 | 120 | 580 | 96 | - |
| under 0.3 psi: | | | | | | |
| average space ratio (%) of absorbent structure | 26 | 25 | 26 | 37 | 26 | - |
| average space radius (µm) of absorbent structure | 59 | 87 | 63 | 280 | 45 | - |
| | | | | | | |
| capillary absorption capacity (g/g) at 20 cm | 15 | 11 | 16 | 5 | 16 | - |
| | | | | | | |

[Table 1] (part 2 of 2)   (continued)

|  | Example 1 | Example 2 | Example 3 | Comparative Example 1 | Comparative Example 2 | Comparative Example 3 |
|---|---|---|---|---|---|---|
| under 0.3 psi: |  |  |  |  |  |  |
| fixation ratio (%) of swollen gel | 95 | 100 | 95 | 100 | 80 | 0 |
|  |  |  |  |  |  |  |
|  |  |  |  |  |  |  |
|  | Model absorbent article (1) | Model absorbent article (2) | Model absorbent article (3) | Comparative model absorbent article (1) | Comparative model absorbent article (2) | - |
| Model absorbent article: |  |  |  |  |  |  |
| liquid permeation time (sec): |  |  |  |  |  |  |
| first-time | 77 | 3 | 33 | 24 | 96 |  |
| second-time | 86 | 16 | 54 | 12 | 300 | - |
| third-time | 88 | 17 | 60 | 6 | 270 | - |
| fourth-time | 94 | 23 | 60 | 6 | 235 | - |
|  |  |  |  |  |  |  |
| area (%) of diffusion: |  |  |  |  |  |  |
| first-time | 39 | 60 | 54 | 36 | 31 | - |
| second-time | 50 | 60 | 62 | 42 | 51 | - |
| third-time | 63 | 61 | 68 | 51 | 67 | - |
| fourth-time | 78 | 78 | 73 | 63 | 83 | - |
|  |  |  |  |  |  |  |
| wet-back quantity (g) | 5.4 | 8.4 | 12.6 | 18.5 | 11.5 | - |
|  |  |  |  |  |  |  |

[Table 2] (part 1 of 2)

| | Example 5 | Example 6 | Example 7 | Comparative Example 4 | Comparative Example 5 | Comparative Example 6 |
|---|---|---|---|---|---|---|
| Performances of water-absorbent resin used: | Water-absorbent resin (4) | Water-absorbent resin (5) | Water-absorbent resin (4) | Water-absorbent resin (3) | Water-absorbent resin (6) | Water-absorbent resin (6) |
| capillary absorption capacity (g/g) at 0 cm | 41.4 | 42.9 | 341.4 | 37.9 | 37.4 | 37.4 |
| capillary absorption capacity D1 (g/g) at 20 cm | 24.5 | 29.6 | 24.5 | 6.3 | 10.8 | 10.8 |
| capillary absorption capacity D2 (g/g) at 40 cm | 7.5 | 18.9 | 7.5 | 1.8 | 2.8 | 2.8 |
| | | | | | | |
| absorption capacity (g/g) | 27.2 | 29.6 | 27.2 | 25 | 26 | 26 |
| absorption capacity (g/g) under load (0.3 psi) | 29.1 | 29 | | 27 | 27 | 27 |
| absorption capacity (g/g) under load (0.7 psi) | 22.5 | 19.1 | | 21 | 21 | 21 |
| | | | | | | |
| without load: | | | | | | |
| average space ratio (%) of swollen gel | 54.9 | 55.4 | 54.9 | 53.0 | 45.9 | 45.9 |
| average space radius (μm) of swollen gel | 193 | 162 | 193 | 420 | 207 | 207 |
| under 0.3 psi: | | | | | | |
| average space ratio (%) of swollen gel | 36.6 | 35.9 | 36.6 | 34 | 35 | 35 |
| average space radius (μm) of swollen gel | 73 | 54 | 73 | 138 | 135 | 135 |
| | | | | | | |

[Table 2] (part 2 of 2)

| | Example 5 | Example 6 | Example 7 | Comparative Example 4 | Comparative Example 5 | Comparative Example 6 |
|---|---|---|---|---|---|---|
| average particle diameter (μm) | 218 | 125 | 218 | 500 | 425 | 500 |
| particle diameter distribution (%): | | | | | | |
| 850μm < | 0 | 0 | 0 | 0 | 0 | 0 |
| 500-850 μm | 0 | 0 | 0 | 52 | 37 | 52 |
| 300-500 μm | 25 | 0 | 25 | 36 | 43 | 36 |
| 150-300 μm | 53 | 31 | 53 | 12 | 19 | 12 |
| 75-150 μm | 18 | 55 | 18 | 0 | 1 | 0 |
| 45-75 μm | 3 | 12 | 3 | 0 | 0 | 0 |
| < 45 μm | 1 | 2 | 1 | 0 | 0 | 0 |
| Performances of model absorbent article: | | | | | | |
| maximum absorption quantity (g) | 357 | 368 | 403 | 255 | 263 | 277 |
| | | | | | | |
| liquid permeation rate (ml/sec) | 21.4 | 17.7 | 33.3 | 18.1 | 23.1 | 21.4 |
| | | | | | | |
| whitening time (ml/min): | | | | | | |
| third-time | 16.7 | 14.5 | 17.3 | Undried | Undried | Undried |
| | | | | | | |
| area (%) of diffusion | 80 | 74 | 82 | 63 | 65 | 66 |
| | | | | | | |

As is evident from Table 2, the absorbent articles of Examples 5 to 7 display excellent liquid-diffusing ability and high total absorption quantity, whereas the absorbent articles of Comparative Examples 4 to 6 are inferior in one of the absorption rate, the diffusing ability, and the maximum absorption quantity test. Thus, the effects of the present invention have been confirmed.

INDUSTRIAL APPLICATION

The present invention can provide an absorbent structure, its production process, and an absorbent article comprising the absorbent structure, wherein the absorbent structure has a high water-absorbent resin content and is excellent in the absorption quantity, the absorption rate, and the liquid-diffusing ability, and is used for absorbent articles such as thin type sanitary materials, and wherein, in the absorbent structure, a water-absorbent resin is well fixed to a base material, and the restrictions, due to this fixation, on the swelling are small, and the absorbent structure is excellent also in absorption properties such as capillary absorption capacity and realizes the thinning and weight-lightening so as to be used most suitably for absorbent articles.

Furthermore, the present invention can provide an absorbent structure, its production process, and an absorbent article comprising the absorbent structure, wherein the absorbent structure avoids the problem such that, as to absorbent articles such as thin type sanitary materials having an increased water-absorbent resin concentration, the water-absorbent resin bulkily swells to put the pressure on bodies with the progress of the absorption of liquids, and wherein the absorbent structure does not greatly change such as thickness of crotch zone even if the liquid absorption quantity increases.

**Claims**

1. An absorbent structure, which comprises a base material, a water-absorbent resin layer, and a hot-melt adhesive, wherein the water-absorbent resin layer includes a water-absorbent resin as an essential material and is fixed to the base material with the hot-melt adhesive;
   with the absorbent structure being **characterized by**:

   being a multi-layered structure including at least the following three layers: a layer of the base material; the water-absorbent resin layer; and the hot-melt adhesive; and
   displaying an average space ratio of 30 to 70 % and an average space radius of 100 to 300 $\mu$m during saturation-swelling with a physiological saline (a 0.9 mass % aqueous NaCl solution) without load.

2. An absorbent structure, which comprises a base material, a water-absorbent resin layer, and a hot-melt adhesive, wherein the water-absorbent resin layer includes a water-absorbent resin as an essential material and is fixed to the base material with the hot-melt adhesive;
   with the absorbent structure being **characterized by**:

   being a multi-layered structure including at least the following three layers: a layer of the base material; the water-absorbent resin layer; and the hot-melt adhesive; and
   displaying an average space ratio of 20 to 50 % and an average space radius of 50 to 130 $\mu$m during saturation-swelling with a physiological saline (a 0.9 mass % aqueous NaCl solution) under a load of 2.07 kPa (0.3 psi).

3. An absorbent structure, which comprises a base material, a water-absorbent resin layer, and a hot-melt adhesive, wherein the water-absorbent resin layer includes a water-absorbent resin as an essential material and is fixed to the base material with the hot-melt adhesive;
   with the absorbent structure being **characterized by**:

   being a multi-layered structure including at least the following three layers: a layer of the base material; the water-absorbent resin layer; and the hot-melt adhesive; and
   displaying a capillary absorption capacity of not less than 10 (g/g) at a height of 20 cm.

4. An absorbent structure according to any one of claims 1 to 3, wherein the hot-melt adhesive is in contact with the water-absorbent resin layer in the shape of a finely fibrous net.

5. An absorbent structure, which comprises a base material, a water-absorbent resin layer, and a hot-melt adhesive, wherein the water-absorbent resin layer includes a water-absorbent resin as an essential material and is fixed to the base material with the hot-melt adhesive;
   with the absorbent structure being **characterized by** being a multi-layered structure including at least the following four layers: a layer of the base material; the water-absorbent resin layer; the hot-melt adhesive; and a member adherent to the hot-melt adhesive.

6. An absorbent structure according to claim 5, wherein the member adherent to the hot-melt adhesive is an anti-sticking material.

7. An absorbent structure according to claim 5, wherein the member adherent to the hot-melt adhesive is a hydrophilic release material.

8. An absorbent structure, which comprises a base material, a water-absorbent resin layer, and a hot-melt adhesive, wherein the water-absorbent resin layer includes a water-absorbent resin as an essential material and is fixed to the base material with the hot-melt adhesive;
    with the absorbent structure being:
    **characterized by** being a multi-layered structure including at least the following three layers: a layer of the base material; the water-absorbent resin layer; and the hot-melt adhesive; and
    further **characterized in that** the mobility of the water-absorbent resin as contained in the water-absorbent resin layer increases in the absorbent structure in proportion as the water-absorbent resin layer absorbs a liquid to increase its swelling ratio.

9. An absorbent structure, which comprises a water-absorbent resin as an essential material;
    with the absorbent structure being **characterized in that** the water-absorbent resin exists in a ratio of not less than 50 mass % in the absorbent structure, wherein the water-absorbent resin displays an average space ratio of 45 to 70 % and an average space radius of 100 to 200 µm as to spaces between particles during saturation-swelling with a physiological saline (a 0.9 mass % aqueous NaCl solution) without load, and further displays a capillary absorption capacity of at least 20 g/g.

10. An absorbent structure, which comprises a water-absorbent resin as an essential material;
    with the absorbent structure being **characterized in that** the water-absorbent resin exists in a ratio of not less than 50 mass % in the absorbent structure, wherein the water-absorbent resin displays an average space ratio of 45 to 70 % and an average space radius of 100 to 200 µm as to spaces between particles during saturation-swelling with a physiological saline (a 0.9 mass % aqueous NaCl solution) without load, and further displays an average space ratio of 20 to 50 % and an average space radius of 50 to 85 µm as to spaces between particles during saturation-swelling with a physiological saline (a 0.9 mass % aqueous NaCl solution) under a load of 2.07 kPa (0.3 psi).

11. An absorbent article, which comprises the absorbent structure as recited in any one of claims 1 to 10.

12. A production process for an absorbent structure, which comprises the steps of: supplying a water-absorbent resin as an essential material to a base material, thereby forming a water-absorbent resin layer; and supplying a hot-melt adhesive to the water-absorbent resin layer, thereby fixing the water-absorbent resin layer to the base material;
    with the production process being **characterized by** using, as the water-absorbent resin, a water-absorbent resin which displays an average space ratio of 30 to 70 % and an average space radius of 100 to 300 µm as to spaces between particles during saturation-swelling with a physiological saline (a 0.9 mass % aqueous NaCl solution) without load.

13. A production process for an absorbent structure, which comprises the steps of: supplying a water-absorbent resin as an essential material to a base material, thereby forming a water-absorbent resin layer; and supplying a hot-melt adhesive to the water-absorbent resin layer, thereby fixing the water-absorbent resin layer to the base material;
    with the production process being **characterized by** using, as the water-absorbent resin, a water-absorbent resin which displays an average space ratio of 20 to 50 % and an average space radius of 50 to 130 µm as to spaces between particles during saturation-swelling with a physiological saline (a 0.9 mass % aqueous NaCl solution) under a load of 2.07 kPa (0.3 psi).

14. A production process for an absorbent structure according to claim 12 or 13, wherein the hot-melt adhesive is brought into contact with the water-absorbent resin layer in the shape of a finely fibrous net.

15. A production process for an absorbent structure, which comprises the steps of: supplying a water-absorbent resin as an essential material to a base material, thereby forming a water-absorbent resin layer; and supplying a hot-melt adhesive to the water-absorbent resin layer, thereby fixing the water-absorbent resin layer to the base material;
    with the production process being **characterized in that**: the hot-melt adhesive has a melt viscosity of 700 to 40,000 mPa·s at 160 °C, and is dispersed into the shape of a web of fine fibers by a non-contact-type melt-blow-spraying method, and the dispersed hot-melt adhesive has an amount of 0.1 to 50 g/m$^2$, an average fiber diameter of 5 to 150 µm, a number-average diameter of 10 to 500 µm as to intervals between fibers, and an elongation of not less than 200 % as to fibers at 25 °C.

**16.** A production process for an absorbent structure according to claim 15, wherein: the melt viscosity is in the range of 2,000 to 7,000 mPa·s, and the elongation is not less than 500 %.

**17.** A production process for an absorbent structure according to claim 15 or 16, wherein the water-absorbent resin has an average particle diameter of 50 to 600 μm.

**18.** A production process for an absorbent structure, which comprises the steps of: supplying a water-absorbent resin as an essential material to a base material, thereby forming a water-absorbent resin layer; and supplying a hot-melt adhesive to the water-absorbent resin layer, thereby fixing the water-absorbent resin layer to the base material; with the production process being **characterized by** further comprising the step of temporarily fixing the water-absorbent resin layer to the base material with a temporarily fixing agent before the step of supplying the hot-melt adhesive.

Fig. 1

Fig. 2

Fig. 3

31

32

33

Fig. 4

Fig. 5

## INTERNATIONAL SEARCH REPORT

| | International application No. |
|---|---|
| | PCT/JP03/00584 |

**A. CLASSIFICATION OF SUBJECT MATTER**
Int.Cl$^7$  A61F13/53

According to International Patent Classification (IPC) or to both national classification and IPC

**B. FIELDS SEARCHED**

Minimum documentation searched (classification system followed by classification symbols)
Int.Cl$^7$  A61F13/15-13/84

Documentation searched other than minimum documentation to the extent that such documents are included in the fields searched

| | | | |
|---|---|---|---|
| Jitsuyo Shinan Koho | 1926-1996 | Toroku Jitsuyo Shinan Koho | 1994-2003 |
| Kokai Jitsuyo Shinan Koho | 1971-2003 | Jitsuyo Shinan Toroku Koho | 1996-2003 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2000-238161 A  (Toyo Eizai Corp.),<br>05 September, 2000 (05.09.00),<br>Full text<br>(Family: none) | 1,4,10,11,<br>12,14 |

☐ Further documents are listed in the continuation of Box C.    ☐ See patent family annex.

| | | |
|---|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family | |

| Date of the actual completion of the international search | Date of mailing of the international search report |
|---|---|
| 23 April, 2003 (23.04.03) | 13 May, 2003 (13.05.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

## INTERNATIONAL SEARCH REPORT

| International application No. |
| --- |
| PCT/JP03/00584 |

**Box I    Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box II    Observations where unity of invention is lacking (Continuation of item 3 of first sheet)**

This International Searching Authority found multiple inventions in this international application, as follows:
1.    Claims 1, 4, 10, 11, 12 and 14 relate, in "a laminate comprising three layers --omitted-- fixed to a substrate" and "a method for producing an absorptive material wherein a water-absorbing resin as an essential raw material is supplied --omitted--", to an absorptive material "which exhibits an average clearance radius of 100 to 300 μm when it is swelled to saturation under no load", a method for producing the absorptive material and an absorptive article comprising said absorptive material.
2.    Claims 2 and 13 relate, in "a laminate comprising three layers --omitted—fixed to a substrate", to an absorptive material "which
   (continued to extra sheet)

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.

4. ☒ No required additional search fees were timely paid by the applicant. Consequently, this international search report is restricted to the invention first mentioned in the claims; it is covered by claims Nos.: 1, 4, 10, 11, 12, 14

**Remark on Protest**    ☐    The additional search fees were accompanied by the applicant's protest.

    ☐    No protest accompanied the payment of additional search fees.

Form PCT/ISA/210 (continuation of first sheet (1)) (July 1998)

**INTERNATIONAL SEARCH REPORT**

International application No.

PCT/JP03/00584

<u>Continuation of Box No.II of continuation of first sheet(1)</u>

exhibits an average clearance radius of 50 to 130 μm when it is swelled to saturation under a load of 2.07 kPa (0.3 psi)", and a method for producing the absorptive material.

3. Claim 3 relates, in "a laminate comprising three layers -- omitted-- fixed to a substrate", to an absorptive material "which exhibits a capillary absorption magnification of 10 (g/g) or more at a height of 20 cm".

4. Claims 5 to 7 relate to "a laminate comprising four layers --omitted-- fixed to a substrate".

5. Claim 8 relates, in "a laminate comprising three layers -- omitted-- fixed to a substrate", to an absorptive material "which exhibits a mobility --omitted-- which enhances as the water-absorbing resin absorbs a greater volume of a solution and has a higher swelling percentage.

6. Claim 9 relates, in "an absorptive material containing a water-absorbing resin as an essential raw material", to an absorptive material "which contains a water-absorbing resin exhibiting-- omitted--under no load, and exhibiting--omitted--under no load, and further has a capillary absorption magnification--omitted--".

7. Claim 10 relates, in "an absorptive material containing a water-absorbing resin as an essential raw material", to an absorptive material "which contains a water-absorbing resin exhibiting-- omitted--under no load, and exhibiting--omitted--under no load, and further exhibiting--omitted-- under a load of 2.07 kPa and exhibiting--omitted-- under a load of 2.07 kPa".

8. Claims 15 to 17 relate, in "a method for producing an absorptive material wherein a water-absorbing resin as an essential raw material is supplied --omitted--", to a method for producing an absorptive material "wherein the hot-melt adhesive is--omitted--".

9. Claim 18 relates, in "a method for producing an absorptive material wherein a water-absorbing resin as an essential raw material is supplied --omitted--", to a method for producing an absorptive material "wherein the water absorbing resin layer is temporarily fixed to the substrate with a temporarily fixing agent before the hot-melt adhesive is supplied".

"A laminate comprising three layers --omitted-- fixed to a substrate", "a method for producing an absorptive material wherein a water-absorbing resin as an essential raw material is supplied -- omitted--", and "an absorptive material containing a water-absorbing resin as an essential raw material", appearing in the above groups of inventions 1 to 9, are known techniques, as are described in, for example, JP 2000-238161 A (Toyo Eizai Corp.), and therefore, are not a technical feature which makes contribution over the prior art, although they are common matters in respective groups of inventions.

Further, no matters except the above-described known techniques in the above groups of inventions 1 to 9 were appreciated to be closely linked by a person skilled in the art at the time of filing the present application.

Accordingly, the above groups of inventions 1 to 9 are not a group of inventions which are so linked as to form a single general inventive concept.

Form PCT/ISA/210 (extra sheet) (July 1998)